# EUROPÄISCHE PATENTSCHRIFT

(11) **EP 2 297 111 B1**
(45) Veröffentlichungstag und Bekanntmachung des Hinweises auf die Patenterteilung: **20.06.2012**
(21) Anmeldenummer: 09738223.8
(22) Anmeldetag: 30.04.2009
(51) Int. Cl.: C07D 231/14, C07D 295/145

(54) **VERFAHREN ZUR HERSTELLUNG HALOGENSUBSTITUIERTER 2-(AMINOMETHYLIDEN)-3-OXOBUTTERSÄUREESTER**
METHOD FOR THE PRODUCTION OF HALOGEN-SUBSTITUTED 2-(AMINOMETHYLIDENE)-3- OXOBUTYRIC ACID ESTERS
PROCÉDÉ DE PRODUCTION D'ESTER D'ACIDE 2-(AMINOMÉTHYLIDÈNE)-3-OXOBUTYRIQUE À SUBSTITUTION HALOGÈNE

(30) Priorität: 02.05.2008 EP 08155611
(43) Veröffentlichungstag der Anmeldung: 23.03.2011
(73) Patentinhaber: BASF SE, 67056 Ludwigshafen (DE)
(72) Erfinder: ZIERKE, Thomas, 67459 Böhl-Iggelheim (DE); MAYWALD, Volker, 67071 Ludwigshafen (DE); RACK, Michael, 69214 Eppelheim (DE); SMIDT, Sebastian Peer, 68723 Oftersheim (DE); KEIL, Michael, 67251 Freinsheim (DE); WOLF, Bernd, 67136 Fußgönheim (DE); KORADIN, Christopher, 67067 Ludwigshafen (DE)
(86) Internationale Anmeldenummer: PCT/EP2009/055285
(87) Internationale Veröffentlichungsnummer: WO 2009/133179

(56) Entgegenhaltungen:
- WO-A-03/051820
- WO-A-2005/042468
- PRYADEINA, M. V. ET AL: "Synthesis and structure of 2-ethoxy- and 2- aminomethylidene 3-fluoroalkyl-3-oxopropionates" RUSSIAN JOURNAL OF ORGANIC CHEMISTRY, CODEN: RJOCEQ; ISSN: 1070-4280, Bd. 43, Nr. 7, 2007, Seiten 945-955, XP002557997

## Beschreibung

Die vorliegende Erfindung betrifft ein Verfahren zur Herstellung halogensubstituierter 2-(Aminomethyliden)-3-oxobutter-säureester sowie deren Umsetzung zu 3-Halomethylpyrazol-4-ylcarbonsäuren und deren Estern. Die vorliegende Erfindung betrifft auch halogensubstituierte 2-(Aminomethyliden)-3-oxobutter-säureester.

Die WO 92/12970 beschreibt (3-Difluormethyl-1-methylpyrazol-4-yl)carboxamide und deren Verwendung als Fungizid. Die Herstellung erfolgt ausgehend von einem 4,4-Difluor-3-oxobuttersäureester, der sukzessive mit Triethylorthoformiat und mit Methylhydrazin umgesetzt wird, wobei man einen 3-Difluormethyl-1-methylpyrazol-4-carbonsäureester erhält. Dieser wird anschließend zur entsprechenden Carbonsäure verseift. Diese wird zum entsprechenden Säurechlorid und anschließend mit einem geeigneten Amin zum entsprechenden Amid umgesetzt. Die Bereitstellung des als Ausgangsverbindung benötigten 4,4-Difluor-3-oxobuttersäureesters ist jedoch vergleichsweise kostspielig und schwierig.

Die WO 2005/044804 beschreibt Alkylester fluormethylsubstituierter heterocyclischer Carbonsäuren sowie deren Herstellung durch Halogenaustausch an entsprechenden chlormethylsubstituierten heterocyclischen Carbonsäureestem unter Verwendung von Fluorierungsmitteln. Die Verwendung von Fluorierungsmitteln ist jedoch kostspielig und stellt spezielle Anforderungen an zu treffende Sicherheitsmaßnahmen und die verwendeten Apparaturen.

Die WO 2005/042468 beschreibt die Herstellung von 2-(Dialkylaminomethyliden)-4,4-dihalo-3-oxobuttersäureester durch Umsetzung von Dialkylaminoacrylsäureestern mit Dihaloessigsäurehalogeniden in Gegenwart einer Base. Um die Bildung von Dihaloketenen zu verhindern werden hier insbesondere wässrige Lösungen von Alkali- und Erdalkalihydroxiden als Base verwendet. Bei dieser Umsetzung werden jedoch keine zufriedenstellenden Ausbeuten erhalten. Die erhaltenen 2-(Dialkylaminomethyliden)-4,4-dihalo-3-oxobuttersäureester werden mit C₁-C₄-Alkylhydrazinen zu den entsprechenden N-alkylierten dihalomethylsubstituierten Pyrazol-4-ylcarbonsäureestern umgesetzt. Bei dieser Umsetzung wird jedoch keine zufriedenstellende Selektivität der 3-Dihalomethylverbindung gegenüber der entsprechenden 5-Dihalomethylverbindung erhalten. Die Anschließende Trennung der beiden entstandenen Isomere ist verhältnismäßig aufwändig.

Der vorliegenden Erfindung liegt somit die Aufgabe zugrunde, ein Verfahren zur Bereitstellung von Ausgangsverbindungen für die Herstellung von 3-Halomethylpyrazol-4-ylcarbonsäureestern und deren Derivaten bereitzustellen, durch das die Ausgangsverbindungen mit geringem Aufwand und mit hohen Ausbeuten erhalten werden. Aus den Ausgangsverbindungen sollen sich weiterhin die 3-Halomethylpyrazol-4-ylcarbonsäureester mit hoher Ausbeute herstellen lassen, wobei eine möglichst hohe Selektivität gegenüber der üblicherweise als Nebenreaktion auftretenden Reaktion zum 5-Halomethylpyrazol-4-ylcarbonsäureester erreicht werden soll.

Überraschenderweise wurde gefunden, dass diese Aufgabe durch ein Verfahren zur Herstellung von Verbindungen der Formel (I) gelöst wird,
worin
- R¹: für C₁-C₆-Alkyl, C₁-C₆-Haloalkyl, C₂-C₆-Alkenyl, C₃-C₁₀-Cycloalkyl oder Benzyl steht, wobei die beiden letztgenannten Reste unsubstituiert sind oder 1, 2 oder 3 Substituenten unabhängig voneinander ausgewählt unter Halogen, CN, Nitro, C₁-C₄-Alkyl, C₁-C₄-Haloalkyl, C₁-C₄-Alkoxy und C₁-C₄-Haloalkoxy aufweisen;
- R² und R³: unabhängig voneinander für C₁-C₆-Alkyl, C₁-C₆-Haloalkyl, C₂-C₆-Alkenyl, C₃-C₁₀-Cycloalkyl oder Benzyl stehen, wobei die beiden letztgenannten Reste unsubstituiert sind oder 1, 2 oder 3 Substituenten unabhängig voneinander ausgewählt unter Halogen, CN, Nitro, C₁-C₄-Alkyl, C₁-C₄-Haloalkyl, C₁-C₄-Alkoxy und C₁-C₄-Haloalkoxy aufweisen; oder
- R²: gemeinsam mit R³ und dem Stickstoffatom, an das die beiden Reste gebunden sind, für einen gegebenenfalls substituierten 5- bis 10-gliedrigen heterocyclischen Rest stehen, der neben dem Stickstoffatom weitere 1, 2 oder 3 Heteroatome ausgewählt unter O, N und S als Ringglieder umfassen kann;
- R⁴: für Wasserstoff, Fluor oder Chlor steht; und
- X¹ und X²: unabhängig voneinander für Fluor oder Chlor stehen;

bei dem man eine Verbindung der Formel (II), 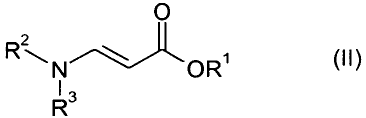 worin R¹, R² und R³ eine der zuvor gegebenen Bedeutungen aufweisen;
mit einer Verbindung der Formel X¹X²R⁴C-C(=O)-F in Gegenwart von wenigstens eines Alkali- oder Erdalkalimetallfluorids umsetzt.

Durch dieses erfindungsgemäße Verfahren lassen sich besonders geeignete Ausgangsverbindungen für ein Verfahren zur Herstellung von 3-Halomethylpyrazol-4-yl)carbonsäureestem mit geringem Aufwand und mit hohen Ausbeuten bereitstellen.

Die hier und im Folgenden in den Formeln der Verbindungen I und II gezeigte E-Konfiguration der C=C-Doppelbindung stellt nur eine mögliche Ausgestaltung der Verbindungen I und II dar. Die Erfindung bezieht sich sowohl auf das gezeigte E-Isomer als auch das Z-Isomer und insbesondere Gemische der Isomere.

Die bei der Definition der Variablen verwendeten Begriffe für organische Gruppen sind, wie beispielsweise der Ausdruck "Halogen", Sammelbegriffe, die stellvertretend für die einzelnen Mitglieder dieser Gruppen organischer Einheiten stehen.

Das Präfix Cₓ-C_{y} bezeichnet im jeweiligen Fall die Anzahl möglicher Kohlenstoffatome.

Der Begriff "Halogen" bezeichnet jeweils Fluor, Brom, Chlor oder Iod, speziell Fluor, Chlor oder Brom.

Der Begriff "C₁-C₆-Alkyl", wie hierin und in den Alkyleinheiten von C₁-C₆-Alkoxy verwendet, bezeichnet eine gesättigte, geradkettige oder verzweigte Kohlenwasserstoffgruppe, umfassend 1 bis 6 Kohlenstoffatome, speziell 1 bis 4 Kohlenstoffatome, beispielsweise Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, 1,1-Dimethylpropyl, 1,2-Dimethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl und deren Isomere. C₁-C₄-Alkyl umfasst beispielsweise Methyl, Ethyl, Propyl, 1-Methylethyl, Butyl, 1-Methylpropyl, 2-Methylpropyl oder 1,1-Dimethylethyl.

Der Begriff "C₁-C₆-Alkoxy" beschreibt geradkettige oder verzweigte gesättigte Alkylgruppen, umfassend 1 bis 6 Kohlenstoffatome, die über ein Sauerstoffatom gebunden sind. Beispiele für C₁-C₆-Alkoxy umfassen, wie Methoxy, Ethoxy, n-Propoxy, 1-Methylethoxy, n-Butoxy, 1-Methylpropoxy, 2-Methylpropoxy, 1,1-Dimethylethoxy, n-Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, n-Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy oder 1-Ethyl-2-methylpropoxy. Beispiele für C₁-C₄-Alkoxy umfassen Methoxy, Ethoxy, n-Propoxy, 1-Methylethoxy, n-Butoxy, 1-Methylpropoxy, 2-Methylpropoxy und 1,1-Dimethylethoxy.

Der Begriff "C₁-C₆-Haloalkyl", wie hierin und in den Haloalkyleinheiten von C₁-C₆-Haloalkoxy verwendet, beschreibt geradkettige oder verzweigte Alkylgruppen mit 1 bis 6 Kohlenstoffatomen, wobei die Wasserstoffatome dieser Gruppen teilweise oder vollständig durch Halogenatome ersetzt sind. Beispiele hierfür sind C₁-C₄-Haloalkyl, wie Chlormethyl, Brommethyl, Dichlormethyl, Trichlormethyl, Fluormethyl, Difluormethyl, Trifluormethyl, Chlorofluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl.

Der Begriff "C₁-C₆-Haloalkoxy" beschreibt geradkettige oder verzweigte gesättigte Haloalkylgruppen, umfassend 1 bis 6 Kohlenstoffatome, die über ein Sauerstoffatom gebunden sind. Beispiele hierfür sind C₁-C₄-Haloalkoxy, wie Chlormethoxy, Brommethoxy, Dichlormethoxy, Trichlormethoxy, Fluormethoxy, Difluormethoxy, Trifluormethoxy, Chlorofluormethoxy, Dichlorfluormethoxy, Chlordifluormethoxy, 1-Chlorethoxy, 1-Bromethoxy, 1-Fluorethoxy, 2-Fluorethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy und Pentafluorethoxy.

Der Begriff "C₂-C₆-Alkenyl" beschreibt geradkettige und verzweigte ungesättigte Kohlenwasserstoffgruppen, umfassend 2 bis 6 Kohlenstoff Atome und wenigstens eine Kohlenstoff-Kohlenstoff-Doppelbindung, wie beispielsweise Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl.

Der Begriff "C₃-C₁₀-Cycloalkyl" wie hierin verwendet, beschreibt mono-, bi- oder tricyclische Kohlenwasserstoffgruppen, umfassend 3 bis 10 Kohlenstoffatome, speziell 3 bis 6 Kohlenstoffatome. Beispiele monocyclischer Gruppen umfassen Cyclopropyl, Cyclobutyl, Cyclopentyl, Cyclohexyl, Cycloheptyl oder Cyclooctyl. Beispiele bicyclischer Gruppen umfassen Bicyclo[2.2.1]heptyl, Bicyclo[3.1.1]heptyl, Bicyclo[2.2.2]octyl und Bicyclo[3.2.1]octyl. Beispiele tricyclischer Gruppen sind Adamantyl und Homoadamantyl.

Der Begriff "5- bis 10-gliedrigen heterocyclischen Rest" steht im Zusammenhang mit der Definition der Gruppe -NR²R³ für eine stickstoffhaltige mono- oder bicyclische Gruppe mit 5, 6, 7, 8, 9 oder 10 Ringgliedern, die über das N-Atom an den übrigen Teil der Verbindung der Formel (I) bzw. (II) gebunden ist, neben dem Stickstoffatom weitere 1, 2 oder 3 Heteroatome ausgewählt unter O, N und S als Ringglieder aufweisen kann und unsubstituiert ist oder 1, 2 oder 3 Substituenten aufweisen kann. Die Substituenten sind, wenn diese an ein Kohlenstoffatom des heterocyclischen Restes gebunden sind, bevorzugt ausgewählt unter Halogen, CN, C₁-C₄-Alkyl, C₁-C₄-HalOalkyl, C₁-C₄-Alkoxy und C₁-C₄-Haloalkoxy, und, wenn diese an ein weiteres Stickstoffatom des heterocyclischen Restes gebunden sind, bevorzugt ausgewählt unter C₁-C₄-Alkyl und C₁-C₄-Haloalkyl. Beispiele 5- bis 10-gliedriger heterocyclischer Reste sind Pyrrol-1-yl, Pyrrolidin-1-yl, Oxazolidin-3-yl, Thiazolidin-3-yl, Imidazol-1-yl, Imidazolin-1-yl, 3-Methylimidazolin-1-yl, 3-Ethylimidazolin-1-yl, 3-Propylimidazolin-1-yl, 3-(1-Methylethyl)imidazolin-1-yl, 3-Butylimidazolin-1-yl, 3-(1,1-Dimethylethyl)imidazolin-1-yl, Pyrazol-1-yl, Pyrazolidin-1-yl, 2-Methylpyrazolidin-1-yl, 2-Ethylpyrazolidin-1-yl, 2-Propylpyrazolidin-1-yl, 2-(1-Methylethyl)pyrazolidin-1-yl, 2-Butylpyrazolidin-1-yl, 2-(1,1-Dimethylethyl)pyrazolidin-1-yl, Piperidin-1-yl, Morpholin-4-yl, Thiamorpholin-4-yl, Piperazin-1-yl, 4-Methylpiperazin-1-yl, 4-Ethylpiperazin-1-yl, 4-Propylpiperazin-1-yl, 4-(1-Methylethyl)piperazin-1-yl, 4-Butylpiperazin-1-yl, 4-(1,1-Dimethylethyl)piperazin-1-yl, Indol-1-yl, Indolin-1-yl, Isoindol-1-yl, Isoindolin-1-yl, Indazol-1-yl, Indazolin-1-yl, 2-Methylindazolin-1-yl, Indazolin-2-yl und 1-Methylindazolin-1-yl, wobei die zuvor genannten heterocyclischen Gruppen unsubstituiert sind oder 1, 2 oder 3 der Ringkohlenstoffatome einen Substituenten ausgewählt unter Halogen, CN, Nitro, C₁-C₄-Alkyl, C₁-C₄-Haloalkyl, C₁-C₄-Alkoxy und C₁-C₄-Haloalkoxy tragen.

Die Kohlenstoff-Kohlenstoff-Doppelbindung in den Verbindungen der Formeln (I) und (II) kann E- oder Z-Konfiguration aufweisen (bzw. cis- oder trans-Konfiguration, bezogen auf die relative Anordnung der Gruppe NR²R³ und der Gruppe -C(O)OR¹).

Die hierin beschriebenen Umsetzungen werden in für derartige Reaktionen üblichen Reaktionsgefäßen durchgeführt, wobei die Reaktionsführung sowohl kontinuierlich als auch diskontinuierlich ausgestaltet werden kann. In der Regel wird man die jeweiligen Reaktionen drucklos durchführen. Die Reaktionen können jedoch auch unter Druck durchgeführt werden.

In den Verbindungen der Formeln (I) und (II) steht R¹ bevorzugt für C₁-C₆-Alkyl, C₃-C₁₀-Cycloalkyl oder Benzyl, wobei die beiden letztgenannten Reste unsubstituiert sind oder 1, 2 oder 3 Substituenten aufweisen. Bevorzugt steht R¹ in den Verbindungen der Formel (I) und (II) für C₁-C₄-Alkyl, C₃-C₆-Cycloalkyl oder Benzyl. Ganz besonders bevorzugt steht R¹ in den Verbindungen der Formeln (I) und (II) für C₁-C₄-Alkyl.

In den Verbindungen der Formeln (I) und (II) stehen R² und R³ unabhängig voneinander bevorzugt für C₁-C₄-Alkyl oder R² steht gemeinsam mit R³ und dem Stickstoffatom, an das die beiden Reste gebunden sind, für einen gegebenenfalls substituierten 5- bis 10-gliedrigen heterocyclischen Rest stehen, der neben dem Stickstoffatom weitere 1, 2 oder 3 Heteroatome ausgewählt unter O, N und S als Ringglieder umfassen kann.

Besonders bevorzugt stehen R² und R³ gemeinsam mit dem Stickstoffatom, an das die beiden Reste gebunden sind, für einen gegebenenfalls substituierten 5- bis 10-gliedrigen heterocyclischen Rest, der neben dem Stickstoffatom weitere 1, 2 oder 3 Heteroatome ausgewählt unter O, N und S als Ringglieder umfassen kann, d. h. die Gruppe NR²R³ steht für ein N-gebundenen 5- bis 10-gliedrigen heterocyclischen Rest. Diese Bevorzugung bezieht sich sowohl auf die Verbindungen der Formeln (I) und (II) an sich, als auch auf deren Verwendung in den erfindungsgemäßen Verfahren zur Herstellung von 3-Halomethylpyrazol-4-ylcarbonsäureestern.

In den Verbindungen der Formeln (I) und (II) steht die Gruppe NR²R³ ganz besonders bevorzugt für einen gesättigten, gegebenenfalls substituierten, 5- oder 6-gliedrigen heterocyclischen Rest, der neben dem Stickstoffatom ein weiteres Heteroatom ausgewählt unter O, N und S als Ringglieder aufweisen kann. Insbesondere steht die Gruppe NR²R³ für Pyrrotidin-1-yl, Oxazolidin-3-yl, 3-Methylimidazolin-1-yl, Piperidin-1-yl, Morpholin-4-yl oder 4-Methylpiperazin-1-yl. Speziell steht die Gruppe NR²R³ in den Verbindungen der Formeln (I) und (II) für Piperidin-1-yl, Morpholin-4-yl oder 4-Methylpiperazin-1-yl.

In den Verbindungen der Formeln (I) und (II) und folglich auch in den Verbindungen der Formel X¹X²R⁴C-C(=O)-F steht R⁴ bevorzugt für Wasserstoff oder Fluor. Besonders bevorzugt steht R⁴ für Wasserstoff.

In den Verbindungen der Formeln (I) und (II) und folglich auch in den Verbindungen der Formel X¹X²R⁴C-C(=O)-F stehen X¹ und X² bevorzugt für Fluor.

Geeignete Verbindungen der Formel X¹X²R⁴C-C(=O)-F sind beispielsweise Difluoracetylfluorid, Dichloracetylfluorid, Trifluoracetylfluorid, Difluorchloracetylfluorid, Dichlorfluoracetylfluorid und Trichloracetylfluorid. Bevorzugte Verbindungen der Formel X¹X²R⁴C-C(=O)-F sind Difluoracetylfluorid, Dichloracetylfluorid, Trifluoracetylfluorid und Difluorchloracetylfluorid. Besonders bevorzugte Verbindungen der Formel X¹X²R⁴C-C(=O)-F sind Difluoracetylfluorid, Difluorchloracetylfluorid und Trifluoracetylfluorid. Ganz besonders bevorzugt wird in dem erfindungsgemäßen Verfahren Difluoracetylfluorid verwendet.

Üblicherweise setzt man in dem erfindungsgemäßen Verfahren zur Herstellung von Verbindungen der Formel (I) die Verbindung der Formel (II) in einer Menge von 0,2 bis 3 Mol, bevorzugt von 0,3 bis 1,5 Mol, besonders bevorzugt von 0,5 bis 1,0 Mol, speziell 0,9 bis 1,0 Mol, jeweils bezogen auf 1 Mol der Verbindung der Formel X¹X²R⁴C-C(=O)-F, ein.

Die Umsetzung erfolgt, indem man die Ausgangsverbindungen, d. h. die Verbindung der Formel (II) und die Verbindung der Formel X¹X²R⁴C-C(=O)-F, vorzugsweise in einem geeigneten Lösungsmittel in einem Reaktionsgefäß miteinander in Kontakt bringt, wobei man in der Regel die Verbindung der Formel (II) und gegebenenfalls das Lösungsmittel im Reaktionsgefäß vorlegt.

Üblicherweise führt man die Umsetzung der Verbindung der Formel (II) mit der Verbindung der Formel X¹X²R⁴C-C(=O)-F bei einer Temperatur im Bereich von -70 bis +50 °C, bevorzugt von -30 bis +20 °C und besonders bevorzugt von -10 bis 0 °C, durch. In einer speziellen Ausführungsform wird zu Beginn eine Temperatur von -50 bis -10 °C eingestellt und im Verlauf der Reaktion auf +10 bis +40 °C, insbesondere Raumtemperatur, erhöht.

Üblicherweise führt man die Umsetzung der Verbindung der Formel (II) mit der Verbindung der Formel X¹X²R⁴C-C(=O)-F bei Atmosphärendruck durch. Aufgrund des niedrigen Siedepunkts der Verbindung der Formel X¹X²R⁴C-C(=O)-F kann es jedoch in Abhängigkeit der gewählten Reaktionstemperatur von Vorteil sein die Umsetzung unter höherem Druck durchzuführen. Geeignete Reaktionsdrücke liegen beispielsweise in einem Bereich von 0,5 bis 10 bar. Geeignete druckfeste Reaktoren sind dem Fachmann ebenfalls bekannt und werden z. B. in Ullmanns Enzyklopädie der technischen Chemie, Bd. 1, 3. Auflage, 1951, S. 769 ff. beschrieben.

Vorzugsweise erfolgt in dem erfindungsgemäßen Verfahren zur Herstellung von Verbindungen der Formel (I) die Umsetzung der Verbindung der Formel (II) mit der Verbindung der Formel X¹X²R⁴C-C(=O)-F im Wesentlichen wasserfrei, d. h. in einem trockenen organischen Lösungsmittel.

Hier und im Folgenden bedeutet trockenes Lösungsmittel, dass das Lösungsmittel einen Wassergehalt von weniger als 500 ppm und insbesondere von weniger als 100 ppm aufweist.

Beispiele geeigneter organischer Lösungsmittel sind unpolare, aprotischen Lösungsmitteln, z.B. aromatische Kohlenwasserstoffe, wie Benzol, Toluol, Xylole, oder (cyclo)aliphatische Kohlenwasserstoffe, wie Hexan, Cyclohexan und dergleichen, sowie Mischungen der zuvor genannten Lösungsmittel.

Beispiele geeigneter organischer Lösungsmittel sind ebenso aprotische polare Lösungsmittel, z. B. cyclische oder acyclische Ether, wie Diethylether, tert.-Butylmethylether (MTBE), Diisopropylether, Cyclopentylmethylether, Tetrahydrofuran (THF) oder Dioxan, cyclische oder acyclische Amide, wie Dimethylformamid, Dimethylacetamid, N-Methylpyrrolidon, Harnstoffe, wie N,N'-Dimethyl-N,N'-ethylenharnstoff (DMEU), N,N'-Dimethyl-N,N'-propylenharnstoff (DMPU) oder Tetramethylharnstoff, oder aliphatische Nitrile, wie Acetonitril oder Propionitril, sowie Mischungen der zuvor genannten Lösungsmittel.

Ebenso geeignet sind Mischungen der vorgenannten unpolaren, aprotischen organischen Lösungsmittel mit polaren, aprotischen Lösungsmitteln.

Erfindungsgemäß wird die Verbindung der Formel (II) in Gegenwart wenigstens eines Alkali- oder Erdalkalimetallfluorids umgesetzt. In der Regel wird man die Verbindung der Formel (II) in Gegenwart wenigstens äquimolarer Mengen wenigstens eines Alkali-oder Erdalkalimetallfluorids, bezogen auf die Verbindung der Formel (II), umsetzen, d. h. in einer Menge von wenigstens einem Mol, bezogen auf ein Mol der Verbindung (II). Bevorzugt wird man die Verbindung der Formel (II) in Gegenwart von 1,1 bis 5 Mol, besonders bevorzugt von 1,2 bis 2 Mol wenigstens eines Alkali- oder Erdalkalimetallfluorids, jeweils bezogen auf 1 Mol der Verbindung der Formel (II), umsetzen.

Geeignete Alkali- oder Erdalkalimetallfluoride sind beispielsweise Lithiumfluorid (LiF), Natriumfluorid (NaF), Kaliumfluorid (KF), Magnesiumfluorid (MgF₂) oder Calciumfluorid (CaF₂, Flussspat) oder deren Gemische. Diese können ebenso in kristalliner Form, wie auch amorpher Form oder auch in technisch hergestellter Form, wie beispielsweise in sprühgetrockneter Form, verwendet werden. Bevorzugt werden in dem erfindungsgemäßen Verfahren NaF, KF oder MgF₂ oder deren Gemische verwendet. Besonders bevorzugt wird in dem erfindungsgemäßen Verfahren KF verwendet.

Üblicherweise wird man die Verbindung der Formel (II) mit der Verbindung der Formel X¹X²R⁴C-C(=O)-F ohne Zusatz einer von dem Alkali- oder Erdalkalimetallfluorid und der Verbindung der Formel (II) verschiedenen weiteren Base umsetzen.

Es ist jedoch ebenso möglich die Verbindung der Formel (II) mit der Verbindung der Formel X¹X²R⁴C-C(=O)-F in Gegenwart einer zusätzlichen Base umzusetzen.

Als zusätzliche Basen eignen sich organische Basen, z. B. acyclische tertiäre Amine, z.B. Tri-C₁-C₆-alkylamine wie Trimethylamin, Triethylamin, Diisopropylethylamin, tert.-Butyldimethylamin, N-C₃-C₆-cycloalkyl-N,N-di-C₁-C₆-alkylamine oder N,N-bis-C₃-C₆-cycloalkyl-N-C₁-C₆-alkylamine wie Ethyldicyclohexylamin, cyclische tertiäre Amine, z.B. N-C₁-C₆-Alkyl-Stickstoffheterocyclen wie N-Methylpyrrolidin, N-Methylpiperidin, N-Methylmorpholin, N,N'-Dimethylpiperazin, Pyridinverbindungen wie Pyridin, Collidin, Lutidin oder 4-Dimethylaminopyridin, sowie bicyclische Amine, wie Diazabicycloundecen (DBU) oder Diazabicyclononen (DBN).

Als zusätzliche Basen eignen sich ebenso anorganische Verbindungen, z.B. Alkalimetall- und Erdalkalimetallcarbonate, wie Lithiumcarbonat oder Calciumcarbonat, Alkalimetallhydrogencarbonate, wie Natriumhydrogencarbonat, Alkalimetall- und Erdalkalimetalloxide, wie Lithiumoxid, Natriumoxid, Calciumoxid oder Magnesiumoxid, Alkalimetall- und Erdalkalimetallhydride, wie Lithiumhydrid, Natriumhydrid, Kaliumhydrid oder Calciumhydrid, oder Alkalimetallamide, wie Lithiumamid, Natriumamid oder Kaliumamid.

Wird in dem erfindungsgemäßen Verfahren eine weitere von Alkali- oder Erdalkalimetallfluoriden und der Verbindung der Formel (II) verschiedene Base (Hilfsbase) zugesetzt, kann diese sowohl in etwa äquimolaren Mengen, bezogen auf die Verbindung (II), z.B. in einer Menge von etwa 0,8 bis 1,2 Mol, bezogen auf 1 Mol der Verbindung (II), als auch in katalytischen Mengen, bezogen auf die Verbindung (II), z.B. in einer Menge von etwa 0,001 bis 0,2 Mol, bezogen auf 1 Mol der Verbindung (II), eingesetzt werden. Die zusätzliche Base kann aber auch in großem Überschuss bezogen auf die Verbindung der Formel (II), z.B. als Lösungsmittel eingesetzt werden. In einer bevorzugten Ausführungsform wird die Hilfsbase, wenn überhaupt, in einer Menge von nicht mehr als 0,2 Mol, insbesondere nicht mehr als 0,1 Mol und speziell nicht mehr als 0,05 Mol, bezogen auf 1 Mol der Verbindung (II) eingesetzt.

Üblicherweise wird man die Verbindung der Formel (I) unter etwa pH-neutralen Bedingungen, d.h. bei einem pH-Wert im Bereich von 4 bis 10, oder unter nicht wässrigen Bedingungen isolieren, um eine übermäßige Hydrolyse der Gruppe -C(O)OR¹ zu verhindern.

Eine Isolierung der Verbindungen der Formel (I) ist jedoch für die im Folgenden beschriebene Umsetzung zu dem entsprechenden Pyrazol-4-ylcarbonsäureester nicht erforderlich. Vielmehr hat es sich als vorteilhaft erwiesen, die Verbindung der Formel (I) nicht zu isolieren und diese als Rohprodukt oder in Form des in dem erfindungsgemäßen Verfahren erhaltenen Reaktionsgemisches zu den entsprechenden Pyrazol-4-ylcarbonsäureestern umzusetzen.

Durch das erfindungsgemäßen Verfahren werden die Verbindungen der Formel (I) ausgehend von den Verbindungen der Formel (11) in guten bis sehr guten Ausbeuten erhalten, d.h. in der Regel in Ausbeuten von wenigstens 70 % und häufig von wenigstens 80 %.

Die Verbindungen der allgemeinen Formel (II) sind kommerziell erhältlich oder können analog zu bekannten Verbindungen, wie beispielsweise in Schema 1 oder 2 veranschaulicht, hergestellt werden.

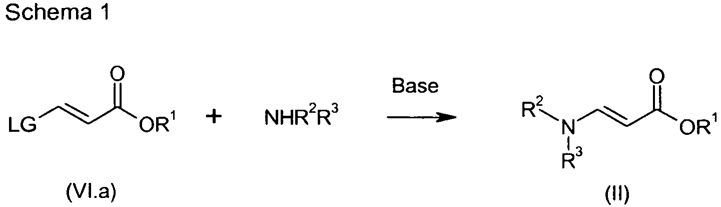

Schema 1 zeigt die Herstellung von Verbindungen der Formel (II) durch Reaktion eines α,β-ungesättigten Esters der Formel (VI.a), worin R¹ eine der zuvor gegebenen Bedeutungen aufweist und LG für eine Abgangsgruppe, wie beispielsweise eine Alkoxygruppe, z.B. C₁-C₆-Alkoxy, mit einem Amin der Formel NHR²R³, worin R² und R³ eine der zuvor gegebenen Bedeutungen aufweisen, in Gegenwart einer Base, wie beispielsweise K₂CO₃. Geeignete Methoden zur Durchführung dieser Reaktion sind dem Fachmann bekannt.

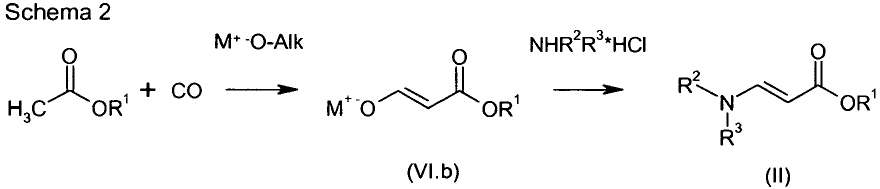

Alternativ lassen sich Verbindungen der Formel (II), analog der EP 0 388 744, durch Umsetzung eines β-Hydroxyacrylsäureestersalzes der Formel (VI.b), worin R¹ eine der zuvor genannten Bedeutungen aufweist und M⁺ beispielsweise für ein Alkalimetallkation, wie Na⁺ oder K⁺, steht, mit einem Ammoniumchlorid der Formel NHR²R³*HCl bereitstellen.

Verbindungen der Formel (VI.b) können beispielsweise durch Umsetzung des entsprechenden Essigsäureesters mit CO und einem Alkoholat der Formel M⁺-O-Alk, worin Alk beispielsweise für C₁-C₄-Alkyl steht, bereitgestellt werden.

Die Amine der Formel NHR²R³ fallen bei der Umsetzung der Verbindungen der Formel (I) zu halomethylsubstituierten Pyrazolylcarbonsäureestern als Nebenprodukt an und können nach erfolgter Umsetzung vorteilhafterweise zurückgewonnen und gegebenenfalls in deren Hydrochloride NHR²R³*HCl überführt werden, um erneut zur Bereitstellung von Verbindungen der Formel (II) gemäß Schema 1 oder 2 verwendet zu werden.

Die durch das erfindungsgemäße Verfahren hergestellten Verbindungen der Formel (I), worin R² gemeinsam mit R³ und dem Stickstoffatom, an das die beiden Reste gebunden sind, für einen heterocyclischen Rest stehen, sind ebenfalls neu. Diese neuartigen Verbindungen der Formel (I) lassen sich mit besonders guten Ausbeuten bereitstellen und eignen sich in besonderem Maß zur Umsetzung mit N-substituierten Hydrazinverbindungen zu 3-Halomethylpyrazol-4-ylcarbonsäureestern, speziell bezüglich Ausbeute und Selektivität des gewünschten Isomers gegenüber dem als Nebenprodukt gebildeten 5-Halomethylpyrazol-4-ylcarbonsäureester.

Die Verbindungen der Formel (I) eignen sich in besonders vorteilhafter Weise zur Herstellung halomethylsubstituierter Pyrazol-4-ylcarbonsäureester.

Demzufolge betrifft ein weiterer Gegenstand der Erfindung ein Verfahren zur Herstellung halomethylsubstituierter Pyrazol-4-ylcarbonsäureester der Formel (III), 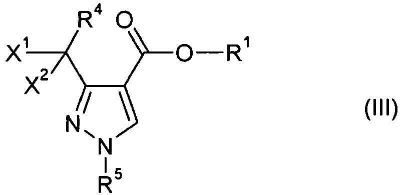 worin
- R¹: für C₁-C₆-Alkyl, C₁-C₆-Haloalkyl, C₂-C₆-Alkenyl, C₃-C₁₀-Cycloalkyl oder Benzyl steht, wobei die beiden letztgenannten Reste unsubstituiert sind oder 1, 2 oder 3 Substituenten unabhängig voneinander ausgewählt unter Halogen, CN, Nitro, C₁-C₄-Alkyl, C₁-C₄-Haloalkyl, C₁-C₄-Alkoxy und C₁-C₄-Haloalkoxy aufweisen;
- R⁴: für Wasserstoff, Fluor oder Chlor steht;
- R⁵: für Wasserstoff, C₁-C₆-Alkyl, C₁-C₆-Haloalkyl, C₃-C₁₀-Cycloalkyl, Phenyl oder Benzyl steht, wobei die drei letztgenannten Reste unsubstituiert sind oder 1, 2 oder 3 Substituenten unabhängig voneinander ausgewählt unter Halogen, CN, Nitro, C₁-C₄-Alkyl, C₁-C₄-Haloalkyl, C₁-C₄-Alkoxy und C₁-C₄-Haloalkoxy aufweisen; und
- X¹ und X²: unabhängig voneinander für Fluor oder Chlor stehen;
umfassend
a) die Bereitstellung einer Verbindung der Formel (I), durch das zuvor beschriebene erfindungsgemäße Verfahren, und
b) die Umsetzung der bereitgestellten Verbindung der Formel (I) mit einer Hydrazin-Verbindung der Formel (IV),

   R⁵HN-NH₂ (IV)

   worin R⁵ eine der zuvor gegebenen Bedeutungen aufweist.

Das erfindungsgemäße Verfahren zur Herstellung von Verbindungen der Formel (III) ist, insbesondere wenn R² und R³ gemeinsam mit dem Stickstoffatom, an das die beiden Reste gebunden sind, für einen heterocyclischen Rest stehen, mit einer Reihe von Vorteilen verbunden. Das erfindungsgemäße Verfahren liefert die Verbindungen der Formel (III) mit hoher Ausbeute. Zudem wird wenn R⁵ eine von H verschiedene Bedeutung aufweist die Verbindung der Formel (III) mit hoher Selektivität gegenüber dem als Nebenprodukt gebildeten 5-Halomethylpyrazol-4-ylcarbonsäureester hergestellt. Somit kann auf eine aufwändige Trennung der Isomerengemische verzichtet oder diese zumindest begrenzt werden. Außerdem lässt sich das erfindungsgemäße Verfahren sowohl wasserfrei als auch in Gegenwart von Wasser unter gleichzeitigem Erhalt zufriedenstellender Ausbeuten und Überschüsse der Verbindung der Formel (III) durchführen.

Bevorzugte weist die Gruppe R¹ in den Verbindungen der Formel (III) eine der Bedeutungen auf, die zuvor als bevorzugte Bedeutungen der Gruppen R¹ in den Verbindungen der Formeln (I) und (II) genannt worden sind.

In einer speziellen Ausführungsform des erfindungsgemäßen Verfahrens, weist die Gruppe R⁵ in den Verbindungen der Formeln (III) und (IV) eine von Wasserstoff verschiedene Bedeutung auf. Die Verbindungen der Formel (III), worin R⁵ eine von Wasserstoff verschiedene Bedeutung aufweist, lassen sich durch das erfindungsgemäße Verfahren mit besonders hoher Selektivität gegenüber den entsprechenden 5-Halomethylpyrazol-4-ylcarbonsäureestern herstellen.

Bevorzugt steht die Gruppe R⁵ in den Verbindungen der Formeln (III) und (IV) für C₁-C₄-Alkyl, C₃-C₆-Cycloalkyl, Phenyl oder Benzyl, wobei die drei letztgenannten Reste unsubstituiert sind oder 1, 2 oder 3 Substituenten aufweisen, die unabhängig voneinander unter Halogen, C₁-C₄-Alkyl und C₁-C₄-Alkoxy ausgewählt sind. Besonders Bevorzugt steht R⁵ in den Verbindungen der Formeln (III) und (IV) für C₁-C₆-Alkyl, insbesondere für C₁-C₄-Alkyl und speziell für Methyl.

In einer speziellen Ausführungsform des erfindungsgemäßen Verfahrens zur Herstellung von Verbindungen der Formel (III) stellt man die Verbindung der Formel (I) in Form eines Reaktionsgemisches bereit, das durch das zuvor beschriebene erfindungsgemäße Verfahren erhalten wurde, ohne die Verbindung der Formel (I) zu isolieren, d.h. die Herstellung der Verbindungen der Formel (III) und deren Umsetzung mit der Hydrazinverbindung der Formel (IV) erfolgt konzertiert in einem sogenannten Eintopfverfahren.

### Schritt b)

Vorzugsweise setzt man die Hydrazinverbindung der Formel (IV), in äquimolaren Mengen oder im Überschuss bezogen auf die Verbindung der Formel (I) ein, wobei ein größerer Überschuss an Verbindung (IV), z. B. mehr als 20 Mol-%, in der Regel nicht erforderlich ist. Vorzugsweise setzt man 1,0 bis 1,2 Mol, insbesondere etwa 1,01 bis 1,15 Mol der Hydrazinverbindung (IV) je Mol der Verbindung (I) ein.

Bevorzugt handelt es sich bei der Hydrazinverbindung der Formel (IV) um ein C₁-C₆-Alkylhydrazin und insbesondere um ein C₁-C₄-Alkylhydrazin; speziell handelt es sich bei der Verbindung der allgemeinen Formel (IV) um Methylhydrazin.

Steht R⁵ in den Verbindungen der Formel (III) für Wasserstoff, verwendet man bevorzugt Hydrazin-Hydrat als Verbindung der Formel (IV).

Bei der Umsetzung der Verbindung der Formel (I) mit der Hydrazinverbindung (IV) wird man üblicherweise so vorgehen, dass man die Hydrazinverbindung der Formel (IV) in einem geeigneten Lösungsmittel vorlegt, die gewünschte Reaktionstemperatur einstellt und anschließend die Verbindung der Formel (I), gegebenenfalls in Form einer Lösung und/oder eines bei der Bereitstellung erhaltenen Reaktionsgemisches, zugibt.

Vorzugsweise legt man die Hydrazinverbindung der Formel (IV) als Lösung in einem organischen Lösungsmittel oder in einem Lösungsmittel/Wasser-Gemisch vor. Alternativ kann man auch so vorgehen, dass man die Hydrazinverbindung der Formel (IV), vorzugsweise als Lösung in einem organischen Lösungsmittel oder in einem Lösungsmittel/Wasser-Gemisch, zu der Verbindung der Formel (I), gegebenenfalls in Form einer Lösung in einem organischen Lösungsmittel oder in einem Lösungsmittel/WasserGemisch, gibt.

Für die Umsetzung der Verbindung der Formel (I) mit der Hydrazinverbindung (IV) geeignete organische Lösungsmittel sind beispielsweise protisch polare Lösungsmittel, wie aliphatische Alkohole mit vorzugsweise 1 bis 4 Kohlenstoffatomen, speziell Methanol, Ethanol, n-Propanol, Isopropanol, n-Butanol, Isobutanol oder tert.-Butanol, unpolare, aprotische Lösungsmittel, z.B. aromatische Kohlenwasserstoffe, wie Benzol, Toluol, Xylole, Mesitylen, Cumol, Chlorbenzol, Nitrobenzol oder tert.-Butylbenzol, aprotische polare Lösungsmittel, wie cyclische oder acyclische Ether, speziell Diethylether, tert.-Butylmethylether (MTBE), Cyclopentylmethylether, Tetrahydrofuran (THF) oder Dioxan, cyclische oder acyclische Amide, speziell Dimethylformamid, Dimethylacetamid, N-Methylpyrrolidon, Harnstoffe, wie N,N'-Dimethyl-N,N'-ethylenharnstoff (DMEU), N,N'-Dimethyl-N,N'-propylenharnstoff (DMPU) oder Tetramethylhamstoff, oder aliphatische Nitrile, speziell Acetonitril oder Propionitril, oder Mischungen der zuvor genannten Lösungsmittel.

Die Umsetzung der Verbindung der Formel (I) mit der Hydrazinverbindung (IV) kann gegebenenfalls in Gegenwart einer Base durchgeführt werden.

Geeignete Basen hierfür sind organische Basen , z. B. die vorgenannten acyclischen tertiären Amine, wie Trimethylamin, Triethylamin, Diisopropylethylamin, tert.-Butyldimethylamin oder Ethyldicyclohexylamin, die vorgenannten cyclischen tertiäre Amine, wie N-Methylpyrrolidin, N-Methylpiperidin, N-Methylmorpholin, N,N'-Dimethylpiperazin, Pyridin, Collidin, Lutidin oder 4-Dimethylaminopyridin, oder bicyclische Amine, wie Diazabicycloundecen (DBU) oder Diazabicyclononen (DBN).

Ebenso als Basen geeignet sind anorganische Verbindungen, z.B. Alkalimetall- und Erdalkalimetallhydroxide, wie Natriumhydroxid, Kaliumhydroxid oder Calciumhydroxid, Alkalimetall- und Erdalkalimetalloxide, wie Lithiumoxid, Natriumoxid, Calciumoxid oder Magnesiumoxid, Alkalimetall- und Erdalkalimetallcarbonate, wie Lithiumcarbonat oder Calciumcarbonat, Alkalimetallhydrogencarbonate, wie Natriumhydrogencarbonat, Alkalimetall- und Erdalkalimetallhydride, wie Lithiumhydrid, Natriumhydrid, Kaliumhydrid oder Calciumhydrid, oder Alkalimetallamide, wie Lithiumamid, Natriumamid oder Kaliumamid.

Die Base kann sowohl in etwa äquimolaren Mengen, bezogen auf die Verbindung (I), z.B. in einer Menge von etwa 0,8 bis 1,2 Mol, bezogen auf 1 Mol der Verbindung (I), als auch in katalytischen Mengen, bezogen auf die Verbindung (I), z.B. in einer Menge von etwa 0,001 bis 0,2 Mol, bezogen auf 1 Mol der Verbindung (I), eingesetzt werden. Die Base kann aber auch in großem Überschuss bezogen auf die Verbindung der Formel (II), z.B. als Lösungsmittel eingesetzt werden.

Durch die Zugabe einer Base können gegebenenfalls größere Überschüsse der 3-Halomethylpyrazol-4-ylcarbonsäureester der Formel (III) bezogen auf die als Nebenprodukt gebildeten 5-Halomethylpyrazol-4-ylcarbonsäureester erzielt werden.

In einer speziellen Ausführungsform des erfindungsgemäßen Verfahrens zur Herstellung von Verbindungen der Formel (III), erfolgt die Umsetzung der Verbindung der Formel (I) mit der Hydrazinverbindung der Formel (IV) in Gegenwart von Wasser. Hierbei ist bereits ein geringer Wassergehalt des Reaktionsgemisches von 1000 ppm ausreichend. Das bei der Umsetzung freigesetzte Wasser ist bei der Angabe des Wassergehalts nicht berücksichtigt.

In der Regel wird der Wassergehalt des Reaktionsgemisches 50 Vol.-%, häufig 30 Vol.-%, insbesondere 15 Vol.-% nicht überschreiten und liegt häufig im Bereich von 0,1 bis 50 Vol.-%, vorzugsweise im Bereich von 0,5 bis 30 Vol.-%, insbesondere im Bereich von 1 bis 15 Vol.-%.

Üblicherweise führt man die Umsetzung der Verbindung der Formel (I) in Gegenwart von Wasser bei Temperaturen von -80 bis +100 °C durch. In einer speziellen Ausführungsform wird zu Beginn der Umsetzung eine Temperatur von -50 bis +20 °C, insbesondere von -15 bis +10 °C, eingestellt und im Verlauf der Reaktion auf eine Temperatur von +10 bis +40 °C, insbesondere auf Raumtemperatur, erhöht.

Führt man die Umsetzung der Verbindung der Formel (I) in Gegenwart von Wasser und einer Base durch, ist die Base vorzugsweise unter den zuvor genannten anorganischen Verbindungen, speziell unter den zuvor genannten Alkalimetall- oder Erdalkalimetallbasen und insbesondere unter Alkalimetallhydroxiden oder Erdalkalimetallhydroxiden, wie NaOH oder KOH, ausgewählt. Bezüglich der eingesetzten Mengen gilt das zuvor Gesagte.

Durch das erfindungsgemäße Verfahren werden die Verbindungen der Formel (III), bei Umsetzung einer Verbindungen der Formel (I) in Gegenwart von Wasser, in guten Ausbeuten erhalten, d.h. in der Regel in Ausbeuten von wenigstens 60 % und häufig von wenigstens 70 %. Zudem werden, wenn R⁵ eine von H verschiedene Bedeutung aufweist, die Verbindungen der Formel (III) in dieser Ausführungsform des Verfahrens mit hoher Selektivität gegenüber den entsprechenden 5-Halomethylpyrazol-4-ylcarbon-säureestern erhalten, d.h. in der Regel in einem Verhältnis von 3-Halomethylpyrazol-4-ylcarbonsäureester zu 5-Halomethylpyrazol-4-ylcarbonsäureester von wenigstens 2,5 : 1 und häufig von wenigstens 5 : 1. Durch die Gegenwart einer geeigneten Base werden häufig Verhältnisse von wenigstens 10 : 1 oder sogar 20 :1 erzielt.

In einer weiteren speziellen Ausführungsform des erfindungsgemäßen Verfahrens zur Herstellung von Verbindungen der Formel (III), erfolgt die Umsetzung der Verbindung der Formel (I) mit der Hydrazinverbindung der Formel (IV) im Wesentlichen wasserfrei, d. h. das Reaktionsgemisch weist einen Wassergehalt von weniger als 500 ppm und insbesondere von weniger als 100 ppm auf. Das bei der Umsetzung freigesetzte Wasser ist bei der Angabe des Wassergehalts nicht berücksichtigt.

Üblicherweise führt man das erfindungsgemäße Verfahren, worin die Umsetzung einer Verbindung der Formel (I) im Wesentlichen wasserfrei erfolgt, bei Temperaturen von -80 bis +100 °C durch. In einer speziellen Ausführungsform wird zu Beginn der Umsetzung eine Temperatur von -80 bis -10 °C, insbesondere von -60 bis -30 °C, eingestellt und im Verlauf der Reaktion auf eine Temperatur von +10 bis +40 °C, insbesondere auf Raumtemperatur, erhöht.

Führt man das erfindungsgemäße Verfahren, worin die Umsetzung einer Verbindung der Formel (I) im Wesentlichen wasserfrei erfolgt, in Gegenwart einer Base durch, ist diese vorzugsweise unter Erdalkalimetall- und Alkalimetallcarbonaten und den zuvor genannten organischen Basen, insbesondere unter den organischen Basen und speziell unter den zuvor genannten Pyridinen und acyclische tertiären Amine, wie Pyridin oder Triethylamin, ausgewählt. Bezüglich der verwendeten Menge gilt das zuvor Gesagte.

Durch das erfindungsgemäße Verfahren, worin die Umsetzung einer Verbindungen der Formel (I) im Wesentlichen wasserfrei erfolgt, werden die Verbindungen der Formel (III) in guten bis sehr guten Ausbeuten erhalten, d.h. in der Regel in Ausbeuten von wenigstens 80 % und häufig von wenigstens 90 %. Zudem werden, wenn R⁵ eine von H verschiedene Bedeutung aufweist, die Verbindungen der Formel (III) in dieser Ausführungsform des Verfahrens mit sehr großer Selektivität gegenüber den entsprechenden 5-Halomethylpyrazol-4-ylcarbonsäureestern erhalten, d.h. in der Regel in einem Verhältnis von 3-Halomethylpyrazol-4-ylcarbonsäureester zu 5-Halomethylpyrazol-4-ylcarbonsäureester von wenigstens 10 : 1 und häufig von wenigstens 20 : 1.

Die Aufarbeitung der erhaltenen Reaktionsgemische und die Isolierung der Verbindung der Formel (III) erfolgt in üblicher Weise, beispielsweise durch Entfernen des Lösungsmittels, z. B. unter vermindertem Druck, durch eine wässrige, extraktive Aufarbeitung oder durch eine Kombination dieser Maßnahmen. Eine weitere Reinigung kann beispielsweise durch Kristallisation oder durch Chromatographie erfolgen. Häufig fällt das Produkt bereits in einer Reinheit an, die weitere Reinigungsverfahren überflüssig macht.

Bei der Umsetzung der Verbindungen der Formel (I) zu halomethylsubstituierten Pyrazolylcarbonsäureestern fallen die Amine der Formel NHR²R³ als Nebenprodukt an. Diese können durch geeignete dem Fachmann bekannte Maßnahmen isoliert und zur Bereitstellung von Verbindungen der Formel (II) verwendet zu werden. Die Isolierung der Amine der Formel NHR²R³ kann beispielsweise durch übliche Trennverfahren, wie Fällung durch Einstellen des pH-Werts oder durch Extraktion, erfolgen.

Die Verbindungen der Formel (III) lassen sich durch Hydrolyse in die entsprechenden halomethylsubstituierten Pyrazol-4-ylcarbonsäuren überführen.

Demzufolge betrifft ein weiterer Gegenstand der Erfindung ein Verfahren zur Herstellung einer Pyrazol-4-carbonsäure der Formel (V), 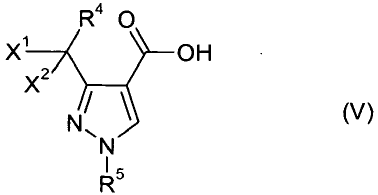 worin R⁴, R⁵, X¹ und X² eine der zuvor gegebenen Bedeutungen besitzt, umfassend die Bereitstellung einer Verbindung der Formel (III), wie zuvor definiert, durch ein erfindungsgemäßes Verfahren und Hydrolyse der Esterfunktion in der bereitgestellten Verbindung der Formel (III), wodurch man die Pyrazolcarbonsäure der Formel (V) erhält.

In einer speziellen Ausführungsform des erfindungsgemäßen Verfahrens zur Herstellung von Verbindungen der Formel (V), wird die Verbindungen der Formel (III) in Form eines durch ein erfindungsgemäßes Verfahren erhaltenen Reaktionsgemisches ohne Isolierung der Verbindung der Formel (III) bereitgestellt, d. h. die Herstellung der Verbindung der Formel (III) und deren Umsetzung zu einer Verbindung der Formel (V) durch Hydrolyse erfolgen konzertiert in einem sogenannten Eintopfverfahren.

Die Hydrolyse der Esterfunktion in der Verbindung (III) kann unter Säurekatalyse oder basisch oder in sonstiger Weise erfolgen. Die Verbindung (III) kann als solche, d. h. nach Isolierung eingesetzt werden. Es ist jedoch auch möglich das in Schritt b) erhaltene Reaktionsgemisch, gegebenenfalls nach Abtrennung flüchtiger Bestandteile, wie Lösungsmittel, ohne Isolierung der Verbindung der Formel (III) umzusetzen.

Bei der basischen Hydrolyse der Verbindung (III) wird man üblicherweise die Verbindung der Formel (III) mit einem Alkalimetallhydroxid oder Erdalkalimetallhydroxid, wie Natriumhydroxid, Kaliumhydroxid oder Lithiumhydroxid, bevorzugt mit einer wässrigen Alkalimetallhydroxid-Lösung oder Erdalkalimetallhydroxid-Lösung, speziell einer wässrigen NaOH-Lösung oder einer wässrigen KOH-Lösung, bis zur vollständigen Hydrolyse des Esters behandeln, vorzugsweise unter Erwärmen.

Bei der basischen Hydrolyse liegt das molare Verhältnis von Verbindung der Formel (III) zu Base üblicherweise im Bereich von 0,8 : 1 bis 1 : 10 und ist insbesondere etwa äquimolar, z.B. im Bereich von 0,8:1 bis 1,2 : 1, jedoch kann auch ein größerer Überschuss an Base, z. B. bis zu 5 Mol je Mol Verbindung (III), von Vorteil sein.

Üblicherweise erfolgt die basische Hydrolyse in einem Verdünnungs- bzw. Lösungsmittel. Geeignete Verdünnungs- bzw. Lösungsmittel sind neben Wasser auch Mischungen organische Lösungsmittel, die gegenüber Alkali stabil sind, mit Wasser. Beispiele für alkalistabile organische Lösungsmittel sind insbesondere die zuvor genannten C₁-C₄-Alkohole sowie die zuvor genannten acyclischen und cyclischen Ether.

Ebenso geeignet sind Gemische unpolarer Lösungsmitteln, z. B. aromatischer Kohlenwasserstoffe, wie Benzol, Toluol, Xylole, oder (cyclo)aliphatischer Kohlenwasserstoffe, wie Hexan, Cyclohexan und dergleichen, mit Wasser.

Vorzugsweise führt man die basische Hydrolyse bei Temperaturen von 20 bis 100 °C durch. In der Regel ist die obere Temperaturgrenze der Siedepunkt des verwendeten Lösungsmittels bei druckloser Reaktionsführung. Vorzugsweise wird man eine Reaktionstemperatur von 100 °C und insbesondere von 90 °C nicht überschreiten. Die Reaktionsdauer ist hierbei von der Reaktionstemperatur, der Konzentration und der Stabilität der jeweiligen Esterverbindung abhängig. Im Allgemeinen werden die Reaktionsbedingungen so gewählt, dass die Reaktionszeit im Bereich von 0,5 bis 12 h und insbesondere im Bereich von 1 bis 6 h liegt.

Die saure Hydrolyse der Estergruppe der Verbindung (III) kann in Analogie zu bekannten sauren Esterhydrolysen durchgeführt werden, d. h. in Gegenwart katalytischer oder stöchiometrischer Mengen einer Säure und Wasser (siehe z. B. J. March, Advanced Organic Chemistry, 2nd Ed., 334-338, McGraw-Hill, 1977 und dort zitierte Literatur). Häufig wird man die Umsetzung in einer Mischung aus Wasser und einem aprotischen organischen Lösungsmittel, beispielsweise einem Ether, wie zuvor genannt, durchführen. Beispiele für geeignete Säuren sind Halogenwasserstoffsäuren, Schwefelsäure, organische Sulfonsäuren, wie p-Toluolsulfonsäure oder Methansulfonsäure, Phosphorsäure sowie saure lonentauscherharze und dergleichen.

Geeignete Hydrolysekatalysatoren sind außerdem Alkalimetalliodide, Lithiumiodid, Trimethyliodsilan oder Mischungen von Trimethylchlorsilan mit Alkalimetalliodiden wie Lithium-, Natrium- oder Kaliumiodid.

Die Isolierung der Säure (V) erfolgt durch übliche Trennverfahren, wie beispielsweise durch Fällung durch Einstellen des pH-Werts oder durch Extraktion.

Bei der Verwendung von Gemischen unpolarer Lösungsmittel mit Wasser als Reaktionsmedium, hat es sich als besonders vorteilhaft erwiesen, die Säure der Formel (V) unter basischen pH-Bedingungen als Lösung in der wässrigen Phase abzutrennen und anschließend durch Einstellen eines sauren pH-Werts aus der wässrigen Lösung als Feststoff auszufällen. Sofern die Verbindungen der Formel (III) in Form eines durch ein erfindungsgemäßes Verfahren bereitgestelltes Reaktionsgemisch ohne vorherige Isolierung der Verbindung der Formel (III) durch Hydrolyse umgesetzt wird, wird bei dieser vorgehensweise das als Nebenprodukt der Vorstufe angefallene Amin der Formel NHR²R³ als Lösung in der abgetrennten organischen Phase erhalten und kann zur Bereitstellung von Verbindungen der Formel (II) verwendet zu werden.

Die Verbindungen der allgemeinen Formel (III) und (V) eignen sich für die Synthese einer Vielzahl als Wirkstoff interessanter Verbindungen, wie beispielsweise zur Herstellung von 3-Halomethylpyrazol-4-carboxamiden, insbesondere von 3-Halomethylpyrazol-4-carboxaniliden.

Geeignete Methoden zur Herstellung von Aniliden durch Umsetzung von Carbonsäuren oder Carbonsäureestern mit aromatischen Aminen sind dem Fachmann bekannt, z.B. aus dem eingangs zitierten Stand der Technik sowie aus J. March, Advanced Organic Chemistry, 2nd Ed., 382 f, McGraw-Hill, 1977 und Organikum, 21. Auflage Wiley-VCH, Weinheim 2001, S. 481-484 und dort zitierte Literatur.

Beispiele für 3-Halomethylpyrazol-4-carboxamide, die sich auf diesem Wege herstellen lassen, sind:
N-(2-Bicyclopropyl-2-yl-phenyl)-3-difluormethyl-1-methylpyrazol-4-yl-carboxamid,
N-(3',4',5'-Trifluorbiphenyl-2-yl)-3-fluormethyl-1-methyl-pyrazol-4-yl-carboxamid,
N-(3',4',5'-Trifluorbiphenyl-2-yl)-3-(chlorfluormethyl)-1-methyl-pyrazol-4-ylcarboxamid,
N-(3',4',5'-Trifluorbiphenyl-2-yl)-3-difluormethyl-1-methyl-pyrazol-4-yl-carboxamid,
N-(3',4',5'-Trifluorbiphenyl-2-yl)-3-(chlordifluormethyl)-1-methyl-pyrazol-4-yl-carboxamid,
N-(3',4',5'-Trifluorbiphenyl-2-yl)-1-methyl-3-trifluormethyl-pyrazol-4-yl-carboxamid,
N-(2',4',5'-Trifluorbiphenyl-2-yl)-3-fluormethyl-1-methyl-pyrazol-4-yl-carboxamid,
N-(2',4',5'-Trifluorbiphenyl-2-yl)-3-(chlorfluormethyl)-1-methyl-pyrazol-4-yl-carboxamid,
N-(2',4',5'-Trifluorbiphenyl-2-yl)-3-difluormethyl-1-methyl-pyrazol-4-yl-carboxamid,
N-(2',4',5'-Trifluorbiphenyl-2-yl)-3-(chlordifluormethyl)-1-methyl-pyrazol-4-yl-carboxamid,
N-(2',4',5'-Trifluorbiphenyl-2-yl)-1-methyl-3-trifluormethyl-pyrazol-4-yl-carboxamid,
N-(3',4'-Dichlor-3-fluorbiphenyl-2-yl)-1-methyl-3-trifluormethyl-1H-pyrazol-4-yl-carboxamid,
N-(3',4'-Dichlor-3-fluorbiphenyl-2-yl)-1-methyl-3-difluormethyl-1 H-pyrazol-4-yl-carboxamid,
N-(3',4'-Difluor-3-fluorbiphenyl-2-yl)-1-methyl-3-trifluormethyl-1H-pyrazol-4-carboxamid,
N-(3',4'-Difluor-3-fluorbiphenyl-2-yl)-1-methyl-3-difluormethyl-1H-pyrazol-4-yl-carboxamid,
N-(3'-Chlor-4'-fluor-3-fluorbiphenyl-2-yl)-1-methyl-3-difluormethyl-1 H-pyrazol-4-yl-carboxamid,
N-(3',4'-Dichlor-4-fluorbiphenyl-2-yl)-1-methyl-3-trifluormethyl-1 H-pyrazol-4-yl-carboxamid,
N-(3',4'-Difluor-4-fluorbiphenyl-2-yl)-1-methyl-3-trifluormethyl-1 H-pyrazol-4-yl-carboxamid,
N-(3',4'-Dichlor-4-fluorbiphenyl-2-yl)-1-methyl-3-difluormethyl-1H-pyrazol-4-yl-carboxamid,
N-(3',4'-Difluor-4-fluorbiphenyl-2-yl)-1-methyl-3-difluormethyl-1 H-pyrazol-4-yl-carboxamid,
N-(3'-Chlor-4'-fluor-4-fluorbiphenyl-2-yl)-1-methyl-3-difluormethyl-1H-pyrazol-4-yl-carboxamid,
N-(3',4'-Dichlor-5-fluorbiphenyl-2-yl)-1-methyl-3-trifluormethyl-1 H-pyrazol-4-yl-carboxamid,
N-(3',4'-Difluor-5-fluorbiphenyl-2-yl)-1-methyl-3-trifluormethyl-1 H-pyrazol-4-yl-carboxamid,
N-(3',4'-Dichlor-5-fluorbiphenyl-2-yl)-1-methyl-3-difluormethyl-1 H-pyrazol-4-yl-carboxamid,
N-(3',4'-Difluor-5-fluorbiphenyl-2-yl)-1-methyl-3-difluormethyl-1H-pyrazole-4-yl-carboxamid,
N-(3'-Chlor-4'-fluor-5-fluorbiphenyl-2-yl)-1-methyl-3-difluormethyl-1 H-pyrazole-4-yl-carboxamid,
N-(4'-Fluor-4-fluorbiphenyl-2-yl)-1-methyl-3-trifluormethyl-1 H-pyrazol-4-yl-carboxamid,
N-(4'-Fluor-5-fluorbiphenyl-2-yl)-1-methyl-3-trifluormethyl-1 H-pyrazole-4-yl-carboxamid,
N-(4'-Chlor-5-fluorbiphenyl-2-yl)-1-methyl-3-trifluormethyl-1H-pyrazol-4-yl-carboxamid,
N-(4'-Methyl-5-fluorbiphenyl-2-yl)-1-methyl-3-trifluormethyl-1H-pyrazol-4-yl-carboxamid,
N-(4'-Fluor-6-fluorbiphenyl-2-yl)-1-methyl-3-trifluormethyl-1H-pyrazol-4-yl-carboxamid,
N-[2-(1,1,2,3,3,3-Hexafluorpropoxy)-phenyl]-3-difluormethyl-1-methyl-1 H-pyrazol-4-yl-carboxamid,
N-[4'-(Trifluormethylthio)-biphenyl-2-yl]-3-difluormethyl-1-methyl-1 H-pyrazol-4-yl-carboxamid,
N-[4'-(Trifluormethylthio)-biphenyl-2-yl]-1-methyl-3-trifluormethyl-1-methyl-1H-pyrazol-4-yl-carboxamid,
3-(Difluormethyl)-1-methyl-N-[1,2,3,4-tetrahydro-9-(1-methylethyl)-1,4-methanonaphthalin-5-yl]-1H-pyrazol-4-yl-carboxamid,
N-(3'-Chlor-5-fluorbiphenyl-2-yl)- 3-(difluormethyl)-1-methylpyrazol-4-yl-carboxamid,
N-(4'-Chlor-5-fluorbiphenyl-2-yl)- 3-(difluormethyl)-1-methylpyrazol-4-yl-carboxamid,
N-(4'-Chlorbiphenyl-2-yl)-3-(difluormethyl)-1-methylpyrazol-4-yl-carboxamid,
N-(4'-Brombiphenyl-2-yl)-3-(difluormethyl)-1-methylpyrazol-4-yl-carboxamid,
N-(4'-Jodbiphenyl-2-yl)-3-(difluormethyl)-1-methylpyrazol-4-yl-carboxamid,
N-(3',5'-difluorbiphenyl-2-yl)-3-(difluormethyl)-1-methylpyrazol-4-yl-carboxamid,
N-(2-Chlor-4-fluor-phenyl)- 3-(difluormethyl)-1-methylpyrazol-4-yl-carboxamid,
N-(2-Brom-4-fluor-phenyl)- 3-(difluormethyl)-1-methylpyrazol-4-yl-carboxamid und
N-(2-Jod-4-fluor-phenyl)- 3-(difluormethyl)-1-methylpyrazol-4-yl-carboxamid.

Im Folgenden wird die vorliegende Erfindung anhand nicht einschränkender Beispiele näher erläutert.

### Beispiele

### 1. Herstellung von Verbindungen der Formel (I)

### Herstellungsbeispiel 1.1: 4,4-Difluor-3-oxo-2-(piperidin-1-ylmethyliden)buttersäure-ethylester

3-Piperidin-1-ylacrylsäureethylester (99%ig, 8,7 g, 50 mmol) und KF (sprühgetrocknet) (4,44 g, 76,3 mmol) wurden in 100 ml Toluol vorgelegt und auf -30°C gekühlt. Anschließend wurde bei dieser Temperatur Difluoracetylfluorid (98%ig, 6,10 g, 61 mmol) eingeleitet. Das Reaktionsgemisch wurde 3h bei -30°C gerührt und anschließend innerhalb einer Stunde auf Raumtemperatur erwärmt. Das Reaktionsgemisch wurde mit vollentsalztem Wasser (50 ml) gewaschen. Nach Trennung der Phasen wurde die wässrige Phase einmal mit Toluol (100 ml) extrahiert. Die Toluolphasen wurden vereinigt, mit einer wässrigen ges. NaCl-Lösung (100 ml) gewaschen und anschließend am Vakuum vom Lösungsmittel befreit. 4,4-Difluor-3-oxo-2-(1-piperidin-1-ylmethyliden)buttersäureethylester wurde als orangefarbenes Öl erhalten (Menge: 13,0 g; Reinheit It. GC: 91,2 %; Ausbeute: 94 %). ¹H-NMR (CDCl₃): δ = 1,3 (t, 3H), 1,75 (m, 6H), 3,3 (m, 2H), 3,6 (m, 2H), 4,25 (q, 2H), 6,6 (t, 1 H), 7,85 ppm (s, 1 H).

### Herstellungsbeispiel 1.2: 4,4,4-Trifluor-3-oxo-2-(piperidin-1-ylmethyliden)buttersäuremethylester

3-Piperidin-1-ylacrylsäuremethylester (97%ig, 25 g, 0,14 mol) und KF (12,5 g, 76,3 mmol) wurden in 250 ml Toluol vorgelegt und auf -30°C gekühlt. Anschließend wurde bei dieser Temperatur Trifluoracetylfluorid (99,5%ig, 18,3 g, 0,16 mol) eingeleitet. Das Reaktionsgemisch wurde 3h bei -30°C gerührt und anschließend innerhalb einer Stunde auf Raumtemperatur erwärmt. Das Reaktionsgemisch wurde mit vollentsalztem Wasser (100 ml) gewaschen. Nach Trennung der Phasen wurde die wässrige Phase einmal mit Toluol (100 ml) extrahiert. Die Toluolphasen wurden vereinigt, mit einer wässrigen ges. NaCl-Lösung (100 ml) gewaschen und anschließend am Vakuum vom Lösungsmittel befreit. 4,4,4-Trifluor-3-oxo-2-(piperidin-1-ylmethyliden)buttersäuremethylester wurde als orangefarbener Feststoff erhalten (Menge: 38,0 g; Reinheit It. GC: 98%; Ausbeute: 98 %). ¹H-NMR (CDCl₃): δ = 1,75 (m, 6H), 3,3 (m, 2H), 3,6 (m, 2H), 3,75 (s, 3H), 7,65 ppm (s, 1 H).

### 2. Herstellung von Verbindungen der Formel (III)

### Herstellungsbeispiel III.1: 3-Difluormethyl-1-methyl-1H-pyrazol-4-ylcarbonsäure-ethylester (Alternative 1)

Methylhydrazin (0,33 g, 7 mmol) wurde in Toluol (50 ml) gelöst und auf -50°C abgekühlt. Bei dieser Temperatur wurde zu dem Reaktionsgemisch eine Lösung von 4,4-Difluor-3-oxo-2-(1-piperidin-1-ylmethyliden)buttersäureethylester (85,5%ig, 2,0 g, 6,5 mmol) in Toluol (50 ml) zugetropft. Das Reaktionsgemisch wurde 2 h bei -50°C gerührt und anschließend innerhalb einer Stunde auf Raumtemperatur erwärmt. Danach wurde das Reaktionsgemisch unter vermindertem Druck vom Lösungsmittel befreit. Der Rückstand wurde in Essigsäureethylester (50 ml) gelöst und mit vollentsalztem (50 ml) Wasser gewaschen. Nach Trocknen der organischen Phase über MgSO₄ wurde das Lösungsmittel unter vermindertem Druck entfernt. 3-Difluormethyl-1-methyl-1 H-pyrazol-4-ylcarbonsäureethylester wurde als Rückstand erhalten (Menge: 1,7 g; Reinheit It. GC: 76,3 % (bezogen auf 3-Isomer); Ausbeute: 97 %). Das Isomerenverhältnis von 3-Isomer zu 5-Isomer im Rückstand betrug 97 : 3. ¹H-NMR (CDCl₃): δ = 1,35 (t, 3H), 3,95 (s, 2H), 4,3 (q, 2H), 7,12 (t, 1 H), 7,9 ppm (s, 1 H).

### Herstellungsbeispiel III.2: 3-Difluormethyl-1-methyl-1H-pyrazol-4-ylcarbonsäure-ethylester (Alternative 2)

Eine wässrige Lösung von Methylhydrazin (35%ig, 0,95 g, 7 mmol) wurde mit Toluol (50 ml) vermischt und auf -50°C abgekühlt. Bei dieser Temperatur wurde zu dem Reaktionsgemisch eine Lösung von 4,4-Difluor-3-oxo-2-(1-piperidin-1-ylmethyliden)buttersäureethylester (85,5%ig, 2,0 g, 6,5 mmol) in Toluol (50 ml) zugetropft. Das Reaktionsgemisch wurde 2 h bei -50°C gerührt und anschließend innerhalb einer Stunde auf Raumtemperatur erwärmt. Danach wurde das Reaktionsgemisch unter vermindertem Druck vom Lösungsmittel befreit. Der Rückstand wurde in Essigsäureethylester (50 ml) gelöst und mit vollentsalztem Wasser (50 ml) gewaschen. Nach Trocknen der organischen Phase über MgSO₄ wurde das Lösungsmittel unter vermindertem Druck entfernt. 3-Difluormethyl-1-methyl-1 H-pyrazol-4-ylcarbonsäureethylester wurde als Rückstand erhalten (Menge: 1,7 g; Reinheit It. GC: 59,3 % (bezogen auf 3-Isomer); Ausbeute: 75,4 %). Das Isomerenverhältnis von 3-Isomer zu 5-Isomer im Rückstand betrug 76 : 24.

### Herstellungsbeispiel III.3: 3-Difluormethyl-1-methyl-1 H-pyrazol-4-ylcarbonsäure-ethylester (Alternative 3)

Eine wässrige Lösung von Methylhydrazin (35%ig, 0,95 g, 7 mmol) wurde mit Triethylamin (0,66 g, 6,5mol) und Toluol (50 ml) vermischt und auf -50°C abgekühlt. Bei dieser Temperatur wurde eine Lösung von 4,4-Difluor-3-oxo-2-(1-piperidin-1-ylmethyliden)-buttersäureethylester (85,5%ig, 2,0 g, 6,5 mmol) in Toluol (50 ml) zugetropft. Das Reaktionsgemisch wurde 2 h bei -50°C gerührt und anschließend innerhalb einer Stunde auf Raumtemperatur erwärmt. Danach wurde das Reaktionsgemisch unter vermindertem Druck vom Lösungsmittel befreit. Der Rückstand wurde in Essigsäureethylester (50 ml) gelöst und mit vollentsalztem Wasser (50 ml) gewaschen. Nach Trocknen der organischen Phase über MgSO₄ wurde das Lösungsmittel unter vermindertem Druck entfernt. 3-Difluormethyl-1-methyl-1H-pyrazolyl-4-ylcarbonsäureethylester wurde als Rückstand erhalten (Menge: 1,8 g; Reinheit It. GC: 64,5 % (bezogen auf 3-Isomer); Ausbeute: 73,4 %). Das Isomerenverhältnis von 3-Isomer zu 5-Isomer im Rückstand betrug 84 : 16.

### Herstellungsbeispiel III.4: 3-Difluormethyl-1-methyl-1 H-pyrazol-4-ylcarbonsäure-ethylester (Alternative 4)

Eine wässrige Lösung von Methylhydrazin (35%ig, 0,95 g, 7 mmol) wurde mit Molekularsieb (5,0 g) und Toluol (50 ml) vermischt und auf -50°C abgekühlt. Bei dieser Temperatur wurde eine Lösung von 4,4-Difluor-3-oxo-2-(1-piperidin-1-ylmethyliden)-buttersäureethylester (85,5%ig, 2,0 g, 6,5 mmol) in Toluol (50 ml) zugetropft. Das Reaktionsgemisch wurde 2 h bei -50°C gerührt und anschließend innerhalb einer Stunde auf Raumtemperatur erwärmt. Danach wurde das Reaktionsgemisch filtriert und unter vermindertem Druck vom Lösungsmittel befreit. Der Rückstand wurde in Essigsäureethylester (50 ml) gelöst und mit vollentsalztem Wasser (50 ml) gewaschen. Nach Trocknen der organischen Phase über MgSO₄ wurde das Lösungsmittel unter vermindertem Druck entfernt. 3-Difluormethyl-1-methyl-1H-pyrazol-4-ylcarbonsäureethylester wurde als Rückstand erhalten (Menge: 1,3 g; Reinheit It. GC: 72,9 % (bezogen auf 3-Isomer); Ausbeute: 71,0 %). Das Isomerenverhältnis von 3-Isomer zu 5-Isomer im Rückstand betrug 88 : 12.

### Herstellungsbeispiel III.5: 3-Difluormethyl-1-methyl-1 H-pyrazol-4-ylcarbonsäure-ethylester (Alternative 5)

Eine wässrige Lösung von Methylhydrazin (35%ig, 1,03 g, 8 mmol) wurde mit Natronlauge (10%ig, 2,6 g, 6,5 mmol) und Ethanol (50 ml) vermischt und auf -3 °C abgekühlt. Bei dieser Temperatur wurde eine Lösung von 4,4-Difluor-3-oxo-2-(1-piperidin-1-ylmethyliden)buttersäureethylester (85,5%ig, 2,0 g, 6,5 mmol) in Toluol (50 ml) zugetropft. Das Reaktionsgemisch wurde 2 h bei -3 °C gerührt und anschließend innerhalb einer Stunde auf Raumtemperatur erwärmt. Danach wurde das Reaktionsgemisch unter vermindertem Druck vom Lösungsmittel befreit. Der Rückstand wurde in Essigsäureethylester (50 ml) gelöst und mit vollentsalztem Wasser (50 ml) gewaschen. Nach Trocknen der organischen Phase über MgSO₄ wurde das Lösungsmittel unter vermindertem Druck entfernt. 3-Difluormethyl-1-methyl-1 H-pyrazol-4-ylcarbonsäureethylester wurde als Rückstand erhalten (Menge: 1,0 g; Reinheit It. GC: 80,6 % (bezogen auf 3-Isomer); Ausbeute: 66,3 %). Das Isomerenverhältnis von 3-Isomer zu 5-Isomer im Rückstand betrug 98 : 2.

### 3. Herstellung von Verbindungen der Formel (V)

### Herstellungsbeispiel V.1: 3-Difluormethyl-1-methyl-1H-pyrazol-4-ylcarbonsäure

Ein Gemisch aus 3-Difluormethyl-1-methyl-1 H-pyrazol-4-ylcarbonsäureethylester (1,4 g, 52 mmol) und Natronlauge (10%ig, 3,1 g, 8 mmol) wurde 2 h bei 60°C gerührt. Das Reaktionsgemisch wurde auf Raumtemperatur abgekühlt und anschließend mit konzentrierter Salzsäure auf pH-Wert von 1 eingestellt. Das Reaktionsgemisch wurde auf 0°C weiter abgekühlt, wobei ein Feststoff ausfiel. Der ausgefallene Feststoff wurde abfiltriert, mit Cyclohexan gewaschen und unter vermindertem Druck getrocknet. 3-Difluormethyl-1-methyl-1 H-pyrazol-4ylcarbonsäure wurde als Feststoff (Menge: 0,8 g; Ausbeute: 87%). ¹H-NMR (DMSO-d₆): δ = 3,9 (s, 2H), 7,2 (t, 1 H), 8,35 ppm (s, 1 H) erhalten.

### Herstellungsbeispiel V.2: 3-Difluormethyl-1-methyl-1 H-pyrazol-4-ylcarbonsäure (konzertierte Fahrweise)

In eine Lösung von 3-Piperidin-1-ylacrylsäuremethylester (99%ig, 58,1 g, 0,34 mol) und KF (sprühgetrocknet) (29,6 g, 0,51 mol) in Toluol (500 ml) wurde bei -30°C Difluoracetylfluorid (37,3 g, 0,373 mol; 98%ig) eingeleitet. Das Reaktionsgemisch wurde 3 h bei - 30 °C gerührt und anschließend innerhalb einer Stunde auf Raumtemperatur erwärmt. Das Reaktionsgemisch wurde mit vollentsalztem Wasser (400 ml) gewaschen. Nach Trennung der Phasen wurde die wässrige Phase einmal mit Toluol (200 ml) extrahiert. Die Toluolphasen wurden vereinigt und am Vakuum auf etwa 600 ml eingeengt. Die so erhaltene Reaktionslösung wurde bei -50 °C zu einer Lösung von Methylhydrazin (17,57 g, 0,373 mol) in Toluol (150 ml) tropfenweise zugegeben. Das Reaktionsgemisch wurde 2 h bei -50°C gerührt und anschließend innerhalb einer Stunde auf Raumtemperatur erwärmt. Danach wurde das Reaktionsgemisch mit wässrigen Natronlauge (10%ig, 210,7 g, 0,527 mol) versetzt und 2 h unter Rückflussbedingungen erhitzt. Nach Abkühlen auf Raumtemperatur wurden die Phasen getrennt. Die Toluolphase wurde mit wässriger Natronlauge (10%ig, 100 ml) extrahiert. Die wässrigen Phasen wurden vereinigt, mit Salzsäure (konz.) auf einen pH-Wert von 1 eingestellt und auf 0°C abgekühlt. Der ausgefallene Feststoff wurde abfiltriert, mit Cyclohexan gewaschen und bei 60°C am Vakuum getrocknet. 3-Difluormethyl-1-methyl-1 H-pyrazol-4-yl-carbonsäure wurde in einer Menge von 56,3 g mit einer Reinheit von 89 % (It. quant. HPLC) erhalten (Ausbeute: 0,285 mol, 85 %). Durch Extraktion des Filtrats mit Ethylacetat wurden weitere 3,5 g eines Feststoffs erhalten, der laut quant. HPLC zu 30 % aus 3-Difluormethyl-1-methyl-1H-pyrazol-4-yl-carbonsäure bestand. ¹H-N MR (CDCl₃): δ = 3,85 (s, 2H), 3,95 (s, 3H), 7,12 (t, 1 H), 7,9 ppm (s, 1 H).

### Herstellungsbeispiel V.3: 3-Difluormethyl-1-methyl-1 H-pyrazol-4-ylcarbonsäure (konzertierte Fahrweise)

In eine Lösung von 3-Piperidin-1-yl-acrylsäuremethylester (99%ig, 29,0 g, 0,17 mol) und KF (technisch) (10,7 g, 0,25 mol) in Toluol (250 ml) wurde bei -30°C Difluoracetylfluorid (18,7 g, 0,187 mol; 98%ig) eingeleitet. Das Reaktionsgemisch wurde 3 h bei - 30 °C gerührt. Anschließend erwärmte man innerhalb einer Stunde auf Raumtemperatur. Das Reaktionsgemisch wurde mit vollentsalztem Wasser (200 ml) gewaschen. Nach Trennung der Phasen wurde die wässrige Phase einmal mit Toluol (100 ml) extrahiert. Die Toluolphasen wurden vereinigt und am Vakuum auf etwa 300 ml eingeengt. Die so erhaltene Reaktionslösung wurde bei -50 °C zu einer Lösung von Methylhydrazin (8,79 g, 0,187 mol) in Toluol (100 ml) tropfenweise zugegeben. Das Reaktionsgemisch wurde 2 h bei -50°C gerührt und anschließend innerhalb einer Stunde auf Raumtemperatur erwärmt. Danach wurde das Reaktionsgemisch mit wässriger Natronlauge (10%ig, 105,3 g, 0,263 mol) versetzt und 2 h unter Rückflussbedingungen erhitzt. Nach Abkühlen auf Raumtemperatur wurden die Phasen getrennt. Die Toluolphase wurde mit wässriger Natronlauge (10%ig, 100 ml) extrahiert. Die wässrigen Phasen wurden vereinigt, mit Salzsäure (konz.) auf einen pH-Wert von 1 eingestellt und auf 0°C abgekühlt. Der ausgefallene Feststoff wurde abfiltriert, mit Cyclohexan gewaschen und bei 60°C am Vakuum getrocknet. 3-Difluormethyl-1-methyl-1H-pyrazol-4-yl-carbonsäure wurde in einer Menge von 29,1 g mit einer Reinheit von 89,4 % (It. quant. HPLC) erhalten (Ausbeute: 88 %). Durch Extraktion des Filtrats mit Ethylacetat wurden weitere 1,4 g eines Feststoffs erhalten, der laut quant. HPLC zu 36 % aus 3-Difluormethyl-1-methyl-1 H-pyrazol-4-yl-carbonsäure bestand.

### Herstellungsbeispiel V.4: 3-Difluormethyl-1-methyl-1H-pyrazol-4-ylcarbonsäure (konzertierte Fahrweise; NaF, technisch)

Der Versuch wurde analog zum vorangegangenen Beispiel jedoch in Gegenwart von NaF als HF-Fänger durchgeführt.

3-Difluormethyl-1-methyl-1 H-pyrazol-4-yl-carbonsäure wurde in einer Menge von 27,5 g mit einer Reinheit von 91,6 % (It. quant. HPLC) erhalten (Ausbeute: 86 %). Durch Extraktion des Filtrats mit Ethylacetat wurden weitere 1,7 g eines Feststoffs erhalten, der laut quant. HPLC zu 40 % aus 3-Difluormethyl-1-methyl-1 H-pyrazol-4-yl-carbonsäure bestand.

### Herstellungsbeispiel V.5: 3-Difluormethyl-1-methyl-1 H-pyrazol-4-ylcarbonsäure (konzertierte Fahrweise; Methylhydrazin, wässrig)

Der Versuch wurde analog zum Beispiel V.3 jedoch in Gegenwart von wässrigem 35%igem Methylhydrazin durchgeführt.

3-Difluormethyl-1-methyl-1 H-pyrazol-4-yl-carbonsäure wurde in einer Menge von 24,1 g mit einer Reinheit von 70,8 % (It. quant. HPLC) erhalten (Ausbeute: 61 % %). Durch Extraktion des Filtrats mit Ethylacetat wurden weitere 5,3 g eines Feststoffs erhalten, der laut quant. HPLC zu 21 % aus 3-Difluormethyl-1-methyl-1H-pyrazol-4-yl-carbonsäure bestand.

### Herstellungsbeispiel V.6: 3-Trifluormethyl-1-methyl-1H-pyrazol-4-ylcarbonsäure (konzertierte Fahrweise)

In eine Lösung von 3-Piperidin-1-ylacrylsäuremethylester (97%ig, 25 g, 0,14 mol) und KF (12,5 g, 0,51 mol) in Toluol (250 ml) wurde bei -30°C Trifluoracetylfluorid (18,3 g, 0,16 mol; 99,5 %ig) eingeleitet. Das Reaktionsgemisch wurde 3 h bei -30 °C gerührt und anschließend innerhalb einer Stunde auf Raumtemperatur erwärmt. Das Reaktionsgemisch wurde mit vollentsalztem Wasser (400 ml) gewaschen. Nach Trennung der Phasen wurde die wässrige Phase einmal mit Toluol (200 ml) extrahiert. Die Toluolphasen wurden vereinigt und am Vakuum auf etwa 600 ml eingeengt. Die so erhaltene Reaktionslösung wurde bei -50 °C zu einer Lösung von Methylhydrazin (7,4 g, 0,16 mol) in Toluol (150 ml) tropfenweise zugegeben. Das Reaktionsgemisch wurde 2 h bei -50°C gerührt und anschließend innerhalb einer Stunde auf Raumtemperatur erwärmt. Danach wurde das Reaktionsgemisch mit wässrigen Natronlauge (10%ig, 69,4 g, 0,17 mol) versetzt und 2 h unter Rückflußbedingungen erhitzt. Nach Abkühlen auf Raumtemperatur wurden die Phasen getrennt. Die Toluolphase wurde mit wässriger Natronlauge (10%ig, 100 ml) extrahiert. Die wässrigen Phasen wurden vereinigt, mit Salzsäure (konz.) auf einen pH-Wert von 1 eingestellt und auf 0°C abgekühlt. Der ausgefallene Feststoff wurde abfiltriert, mit Cyclohexan gewaschen und bei 60°C am Vakuum getrocknet. 3-Trifluormethyl-1-methyl-1 H-pyrazol-4-yl-carbonsäure wurde in einer Menge von 21,8 g mit einer Reinheit von 98 % (It. ¹H-NMR) erhalten (Ausbeute: 0,12 mol, 81 %). ¹H-NMR (CDCl₃): δ = 3,95 (s, 3H), 8,45 ppm (s, 1 H).

## Patentansprüche

1. Verfahren zur Herstellung von Verbindungen der Formel (I), 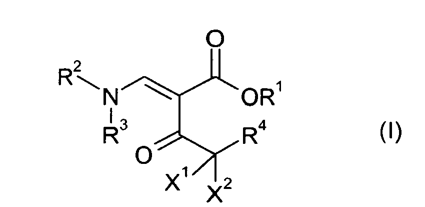 worin
R¹ für C₁-C₆-Alkyl, C₁-C₆-Haloalkyl, C₂-C₆-Alkenyl, C₃-C₁₀-Cycloalkyl oder Benzyl steht, wobei die beiden letztgenannten Reste unsubstituiert sind oder 1, 2 oder 3 Substituenten unabhängig voneinander ausgewählt unter Halogen, CN, Nitro, C₁-C₄-Alkyl, C₁-C₄-Haloalkyl, C₁-C₄-Alkoxy und C₁-C₄-Haloalkoxy aufweisen;
R² und R³ unabhängig voneinander für C₁-C₆-Alkyl, C₁-C₆-Haloalkyl, C₂-C₆-Alkenyl, C₃-C₁₀-Cycloalkyl oder Benzyl stehen, wobei die beiden letztgenannten Reste unsubstituiert sind oder 1, 2 oder 3 Substituenten unabhängig voneinander ausgewählt unter Halogen, CN, Nitro, C₁-C₄-Alkyl, C₁-C₄-Haloalkyl, C₁-C₄-Alkoxy und C₁-C₄-Haloalkoxy aufweisen; oder
R² gemeinsam mit R³ und dem Stickstoffatom, an das die beiden Reste gebunden sind, für einen gegebenenfalls substituierten 5- bis 10-gliedrigen heterocyclischen Rest stehen, der neben dem Stickstoffatom weitere 1, 2 oder 3 Heteroatome ausgewählt unter O, N und S als Ringglieder umfassen kann;
R⁴ für Wasserstoff, Fluor oder Chlor steht; und
X¹ und X² unabhängig voneinander für Fluor oder Chlor stehen;
bei dem man
eine Verbindung der Formel (II), 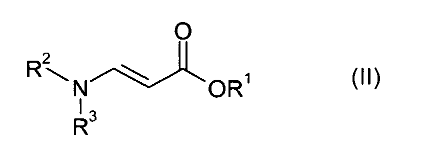 worin R¹, R² und R³ eine der zuvor gegebenen Bedeutungen aufweisen;
mit einer Verbindung der Formel X¹X²R⁴C-C(=O)-F in Gegenwart wenigstens eines Alkali- oder Erdalkalimetallfluorids umsetzt.

2. Verfahren nach Anspruch 1, bei dem man die Verbindung der Formel (II) mit der Verbindung der Formel X¹X²R⁴C-C(=O)-F in Gegenwart wenigstens äquimolarer Mengen wenigstens eines Alkali- oder Erdalkalimetallfluorids, bezogen auf die Verbindung der Formel (II), umsetzt.

3. Verfahren nach einem der vorhergehenden Ansprüche, bei dem man die Verbindung der Formel (II) mit der Verbindung der Formel X¹X²R⁴C-C(=O)-F in Gegenwart von Kaliumfluorid umsetzt.

4. Verfahren nach einem der vorhergehenden Ansprüche, bei dem man die Verbindung der Formel (II) mit Difluoracetylfluorid umsetzt.

5. Verfahren nach einem der vorhergehenden Ansprüche, wobei die Umsetzung der Verbindung der Formel (II) mit der Verbindung der Formel X¹X²R⁴C-C(=O)-F im Wesentlichen wasserfrei erfolgt.

6. Verfahren nach einem der vorhergehenden Ansprüche, wobei R² und R³ in den Verbindungen der Formeln (I) und (II) gemeinsam mit dem Stickstoffatom, an das die beiden Reste gebunden sind, für einen gegebenenfalls substituierten 5- bis 10-gliedrigen heterocyclischen Rest stehen, der neben dem Stickstoffatom weitere 1, 2 oder 3 Heteroatome ausgewählt unter O, N und S als Ringglieder umfassen kann.

7. Verfahren nach Anspruch 6, wobei NR²R³ für einen gesättigten gegebenenfalls substituierten 5- oder 6-gliedrigen heterocyclischen Rest steht, der neben dem Stickstoffatom ein weiteres Heteroatom ausgewählt unter O, N und S als Ringglied aufweisen kann.

8. Verfahren nach Anspruch 7, wobei NR²R³ für Pyrrolidin-1-yl, Oxazolidin-3-yl, 3-Methylimidazolin-1-yl, Piperidin-1-yl, Morpholin-4-yl oder 4-Methylpiperazin-1-yl steht.

9. Verfahren zur Herstellung halomethylsubstituierter Pyrazol-4-ylcarbon-säureester
der Formel (III), 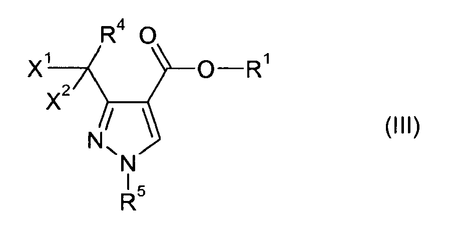 worin
R¹ für C₁-C₆-Alkyl, C₁-C₆-Haloalkyl, C₂-C₆-Alkenyl, C₃-C₁₀-Cycloalkyl oder Benzyl steht, wobei die beiden letztgenannten Reste unsubstituiert sind oder 1, 2 oder 3 Substituenten unabhängig voneinander ausgewählt unter Halogen, CN, Nitro, C₁-C₄-Alkyl, C₁-C₄-Haloalkyl, C₁-C₄-Alkoxy und C₁-C₄-Haloalkoxy aufweisen;
R⁴ für Wasserstoff, Fluor oder Chlor steht;
R⁵ für Wasserstoff, C₁-C₆-Alkyl, C₁-C₆-Haloalkyl, C₃-C₁₀-Cycloalkyl, Phenyl oder Benzyl steht, wobei die drei letztgenannten Reste unsubstituiert sind oder 1, 2 oder 3 Substituenten unabhängig voneinander ausgewählt unter Halogen, CN, Nitro, C₁-C₄-Alkyl, C₁-C₄-Haloalkyl, C₁-C₄-Alkoxy und C₁-C₄-Haloalkoxy aufweisen; und
X¹ und X² unabhängig voneinander für Fluor oder Chlor stehen;
umfassend
a) die Bereitstellung einer Verbindung der Formel (I), durch ein Verfahren gemäß einem der Ansprüche 1 bis 8, und
b) die Umsetzung der bereitgestellten Verbindung der Formel (I) mit einer Hydrazin-Verbindung der Formel (IV),
R⁵HN-NH₂ (IV)
worin R⁵ eine der zuvor gegebenen Bedeutungen aufweist.

10. Verfahren nach Anspruch 9, bei dem R⁵ in den Verbindungen der Formeln (III) und (IV) für C₁-C₄-Alkyl steht.

11. Verfahren nach einem der Ansprüche 9 oder 10, bei dem die Verbindung der Formel (I) in Form eines durch ein Verfahren nach einem der Ansprüche 1 bis 10 erhaltenen Reaktionsgemisches ohne vorherige Isolierung der Verbindung der Formel (I) bereitgestellt wird.

12. Verfahren zur Herstellung von Pyrazol-4-carbonsäuren der Formel (V), 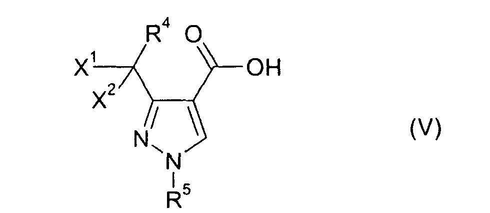 worin R⁴, R⁵, X¹ und X² eine der zuvor gegebenen Bedeutungen aufweisen, umfassend
die Bereitstellung einer Verbindung der Formel (III) durch ein Verfahren gemäß einem der Ansprüche 9 bis 11 und die Umsetzung der bereitgestellten Verbindung der Formel (III) durch Hydrolyse.

13. Verfahren nach Anspruch 12, bei dem man die Verbindung der Formel (III) in Form eines durch ein Verfahren nach einem der Ansprüche 9 bis 11 bereitgestellten Reaktionsgemisches ohne vorherige Isolierung der Verbindung der Formel (III) durch Hydrolyse umsetzt.

14. Verfahren nach einem der Ansprüche 12 oder 13, wobei die Hydrolyse in Gegenwart einer wässrigen Alkalimetallhydroxid-Lösung oder Erdalkalimetallhydroxid-Lösung erfolgt.

## Claims

1. A process for preparing compounds of the formula (I) 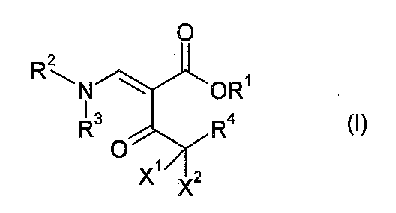 in which
R¹ is C₁-C₆-alkyl, C₁-C₆-haloalkyl, C₂-C₆-alkenyl, C₃-C₁₀-cycloalkyl or benzyl, where the two last mentioned radicals are unsubstituted or have 1, 2 or 3 substituents independently of one another selected from the group consisting of halogen, CN, nitro, C₁-C₄-alkyl, C₁-C₄-haloalkyl, C₁-C₄-alkoxy and C₁-C₄-haloalkoxy;
R² and R³ independently of one another are C₁-C₆-alkyl, C₁-C₆-haloalkyl, C₂-C₆-alkenyl, C₃-C₁₀-cycloalkyl or benzyl, where the two last mentioned radicals are unsubstituted or have 1, 2 or 3 substituents independently of one another selected from the group consisting of halogen, CN, nitro, C₁-C₄-alkyl, C₁-C₄-haloalkyl, C₁-C₄-alkoxy and C₁-C₄-haloalkoxy; or
R² together with R³ and the nitrogen atom to which the two radicals are attached are an optionally substituted 5- to 10-membered heterocyclic radical which, in addition to the nitrogen atom, may contain a further 1, 2 or 3 heteroatoms selected from the group consisting of O, N and S as ring members;
R⁴ is hydrogen, fluorine or chlorine; and
X¹ and X² independently of one another are fluorine or chlorine;
wherein
a compound of the formula (II) 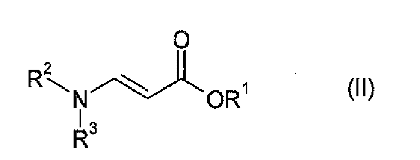 in which R¹, R² and R³ have one of the meanings given above;
is reacted with a compound of the formula X¹X²R⁴C-C(=O)-F in the presence of at least one alkali or alkaline earth metal fluoride.

2. The process according to claim 1 in which the compound of the formula (II) is reacted with the compound of the formula X¹X²R⁴-C-C(=O)-F in the presence of at least equimolar amounts of at least one alkali or alkaline earth metal fluoride, based on the compound of the formula (II).

3. The process according to any of the preceding claims in which the compound of the formula (II) is reacted with the compound of the formula X¹X²R⁴C-C(=O)-F in the presence of potassium fluoride.

4. The process according to any of the preceding claims wherein the compound of the formula (II) is reacted with difluoroacetyl fluoride.

5. The process according to any of the preceding claims where the reaction of the compound of the formula (II) with the compound of the formula X¹X²R⁴C-C(=O)-F is carried out essentially anhydrously.

6. The process according to any of the preceding claims wherein R² and R³ in the compounds of the formulae (I) and (II) together with the nitrogen atom to which the two radicals are attached are an optionally substituted 5- to 10-membered heterocyclic radical which, in addition to the nitrogen atom, may contain a further 1, 2 or 3 heteroatoms selected from the group consisting of O, N and S as ring members.

7. The process according to claim 6 wherein NR²R³ is a saturated optionally substituted, 5- or 6-membered heterocyclic radical which, in addition to the nitrogen atom, may contain a further heteroatom selected from the group consisting of O, N and S as ring member.

8. The process according to claim 7 in which NR²R³ is pyrrolidin-1-yl, oxazolidin-3-yl, 3-methylimidazolin-1-yl, piperidin-1-yl, morpholin-4-yl or 4-methylpiperazin-1-yl.

9. A process for preparing halomethyl-substituted pyrazol-4-ylcarboxylic esters of the formula (III) 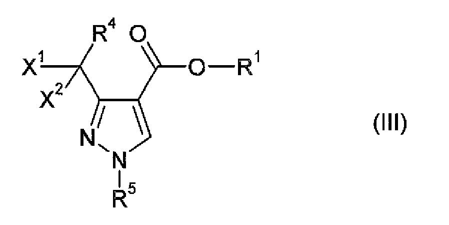 in which
R¹ is C₁-C₆-alkyl, C₁-C₆-haloalkyl, C₂-C₆-alkenyl, C₃-C₁₀-cycloalkyl or benzyl, where the two last mentioned radicals are unsubstituted or have 1, 2 or 3 substituents independently of one another selected from the group consisting of halogen, CN, nitro, C₁-C₄-alkyl, C₁-C₄-haloalkyl, C₁-C₄-alkoxy and C₁-C₄-haloalkoxy;
R⁴ is hydrogen, fluorine or chlorine;
R⁵ is hydrogen, C₁-C₆-alkyl, C₁-C₆-haloalkyl, C₃-C₁₀-cycloalkyl, phenyl or benzyl where the three last mentioned radicals are unsubstituted or have 1, 2 or 3 substituents independently of one another selected from the group consisting of halogen, CN, nitro, C₁-C₉-alkyl, C₁-C₄-haloalkyl, C₁-C₄-alkoxy and C₁-C₄-haloalkoxy; and
X¹ and X² are independently of one another fluorine or chlorine;
comprising
a) the provision of a compound of the formula (I) by means of a process according to one of claims 1 to 8, and
b) the reaction of the compound of the formula (I) provided with a hydrazine compound of the formula (IV)
R⁵HN-NH₂ (IV)
in which R⁵ has one of the meanings given above.

10. The process according to claim 9 wherein R⁵ in the compounds of the formulae (III) and (IV) is C₁-C₄-alkyl.

11. The process according to one of claims 9 or 10 wherein the compound of the formula (I) is provided in the form of a reaction mixture obtained by a process according to any of claims 1 to 10, without prior isolation of the compound of the formula (I).

12. A process for preparing a pyrazol-4-carboxylic acid of the formula (V) 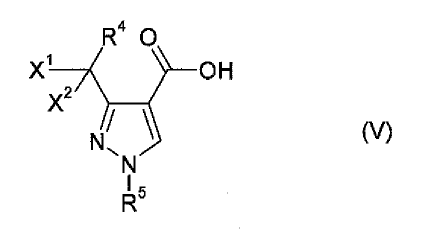 in which R⁹, R⁵, X¹ and X² have one of the meanings given above, comprising
the provision of a compound of the formula (III) by a process according to any of claims 9 to 11 and the hydrolysis of the provided compound of the formula (III).

13. The process according to claim 12 wherein the compound of the formula (III) is hydrolyzed in the form of a reaction mixture provided by a process according to any of claims 9 to 11, without prior isolation of the compound of the formula (III).

14. The process according to one of claims 12 or 13 where the hydrolysis is carried out in the presence of an aqueous alkali metal hydroxide solution or alkaline earth metal hydroxide solution.

## Revendications

1. Procédé pour la préparation de composés de formule (I), 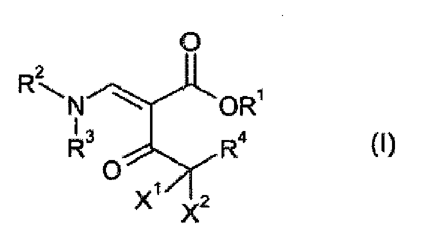 dans laquelle
R¹ représente un groupe alkyle en C₁-C₆, halogénoalkyle en C₁-C₆, alcényle en C₂-C₆, cycloalkyle en C₃-C₁₀ ou benzyle, les deux radicaux nommés en dernier étant non substitués ou comportant 1, 2 ou 3 substituants choisis chacun indépendamment parmi des atomes d'halogène et des groupes CN, nitro, alkyle en C₁-C₄, halogénoalkyle en C₁-C₄, alcoxy en C₁-C₄ et halogénoalcoxy en C₁-C₄ ;
R² et R³ représentent, indépendamment l'un de l'autre, un groupe alkyle en C₁-C₆, halogénoalkyle en C₁-C₆, alcényle en C₂-C₆, cycloalkyle en C₃-C₁₀ ou benzyle, les deux radicaux nommés en dernier étant non substitués ou comportant 1, 2 ou 3 substituants choisis chacun indépendamment parmi des atomes d'halogène et des groupes CN, nitro, alkyle en C₁-C₄, halogénoalkyle en C₁-C₄, alcoxy en C₁-C₄ et halogénoalcoxy en C₁-C₄ ; ou
R² conjointement avec R³ et l'atome d'azote, auquel les deux radicaux sont liés, représentent un radical hétérocyclique à 5-10 chaînons, éventuellement substitué, qui peut comprendre en tant que chaînons formant le cycle, en plus de l'atome d'azote, encore 1, 2 ou 3 hétéroatomes choisis parmi O, N et S ;
R⁴ représente un atome d'hydrogène, de fluor ou de chlore ; et
X¹ et X² représentent, indépendamment l'un de l'autre, un atome de fluor ou de chlore ;
dans lequel on fait réagir
un composé de formule (II), 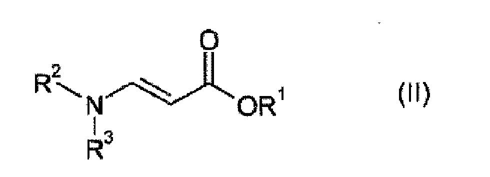 dans laquelle R¹, R² et R³ ont les significations données précédemment ;
avec un composé de formule X¹X²R⁴C-C(=O)-F en présence d'au moins un fluorure de métal alcalin ou alcalino-terreux.

2. Procédé selon la revendication 1, dans lequel on fait réagir le composé de formule (II) avec le composé de formule X¹X²R⁴C-C(=O)-F en présence de quantités au moins équimolaires d'un fluorure de métal alcalin ou alcalino-terreux, par rapport au composé de formule (II).

3. Procédé selon l'une quelconque des revendications précédentes, dans lequel on fait réagir le composé de formule (II) avec le composé de formule X¹X²R⁹C-C(=O)-F en présence de fluorure de potassium.

4. Procédé selon l'une quelconque des revendications précédentes, dans lequel on fait réagir le composé de formule (II) avec du fluorure de difluoroacétyle.

5. Procédé selon l'une quelconque des revendications précédentes, dans lequel on effectue la réaction du composé de formule (II) avec le composé de formule X¹X²R⁴C-C(=O)-F pratiquement en absence d'eau.

6. Procédé selon l'une quelconque des revendications précédentes, dans lequel R² et R³ dans les composés de formules (I) et (II) représentent conjointement avec l'atome d'azote, auquel les deux radicaux sont liés, un radical hétérocyclique à 5-10 chaînons, éventuellement substitué, qui peut comprendre en tant que chaînons formant le cycle, en plus de l'atome d'azote, encore 1, 2 ou 3 hétéroatomes choisis parmi O, N et S.

7. Procédé selon la revendication 6, dans lequel NR²R³ représente un radical hétérocyclique saturé à 5 ou 6 chaînons, éventuellement substitué, qui peut comprendre en tant que chaînon formant le cycle, en plus de l'atome d'azote, encore un autre hétéroatome choisi parmi O, N et S.

8. Procédé selon la revendication 7, dans lequel NR²R³ représente le groupe pyrrolidin-1-yle, oxazolidin-3-yle, 3-méthylimidazolin-1-yle, pipéridin-1-yle, morpholin-4-yle ou 4-méthylpipérazin-1-yle.

9. Procédé pour la préparation d'ester d'acide pyrazol-4-ylcarboxylique substitué par halogénométhyle, de formule (III), 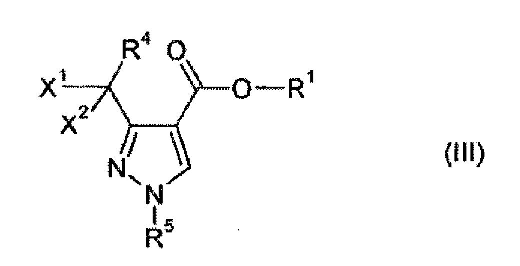 dans laquelle
R¹ représente un groupe alkyle en C₁-C₆, halogénoalkyle en C₁-C₆, alcényle en C₂-C₆, cycloalkyle en C₃-C₁₀ ou benzyle, les deux radicaux nommés en dernier étant non substitués ou comportant 1, 2 ou 3 substituants choisis chacun indépendamment parmi des atomes d'halogène et des groupes CN, nitro, alkyle en C₁-C₄, halogénoalkyle en C₁-C₄, alcoxy en C₁-C₄ et halogénoalcoxy en C₁-C₄ ;
R⁴ représente un atome d'hydrogène, de fluor ou de chlore ;
R⁵ représente un atome d'hydrogène, un groupe alkyle en C₁-C₆, halogénoalkyle en C₁-C₆, cycloalkyle en C₃-C₁₀, phényle ou benzyle, les trois radicaux nommés en dernier étant non substitués ou comportant 1, 2 ou 3 substituants choisis chacun indépendamment parmi des atomes d'halogène et des groupes CN, nitro, alkyle en C₁-C₄, halogénoalkyle en C₁-C₄, alcoxy en C₁-C₄ et halogénoalcoxy en C₁-C₄ ; et
X¹ et X² représentent, indépendamment l'un de l'autre, un atome de fluor ou de chlore ;
comprenant
a) la mise à disposition d'un composé de formule (I) , par un procédé selon l'une quelconque des revendications 1 à 8, et
b) la mise en réaction du composé de formule (I), mis à disposition, avec un composé de type hydrazine de formule (IV),
R⁵HN-NH₂ (IV)
dans laquelle R⁵ a l'une des significations données précédemment .

10. Procédé selon la revendication 9, dans lequel R⁵ dans les composés de formules (III) et (IV) représente un groupe alkyle en C₁-C₄.

11. Procédé selon la revendication 9 ou 10, dans lequel le composé de formule (I) est mis à disposition sous forme d'un mélange réactionnel obtenu par un procédé selon l'une quelconque des revendications 1 à 10, sans isolement préliminaire du composé de formule (I).

12. Procédé pour la préparation d'acides pyrazole-4-carboxyliques de formule (V), 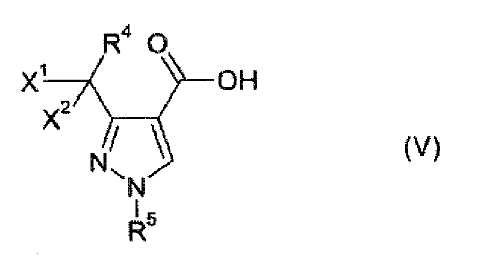 dans laquelle R⁴, R⁵, X¹ et X² ont l'une des significations données précédemment, comprenant
la mise à disposition d'un composé de formule (III) par un procédé selon l'une quelconque des revendications 9 à 11 et la conversion du composé de formule (III), mis à disposition, par hydrolyse.

13. Procédé selon la revendication 12, dans lequel on convertit par hydrolyse le composé de formule (III), sous forme d'un mélange réactionnel mis à disposition par un procédé selon l'une quelconque des revendications 9 à 11, sans isolement préliminaire du composé de formule (III).

14. Procédé selon la revendication 12 ou 13, dans lequel on effectue l'hydrolyse en présence d'une solution aqueuse d'un hydroxyde de métal alcalin ou d'une solution aqueuse d'un hydroxyde de métal alcalino-terreux.
